# EUROPEAN PATENT SPECIFICATION

(11) **EP 1 792 914 B1**
(45) Date of publication and mention of the grant of the patent: **29.07.2009**
(21) Application number: 05026397.9
(22) Date of filing: 02.12.2005
(51) Int. Cl.: C07K 14/52, C07K 14/47, A61K 38/19, C12N 15/62

(54) **Chemokine-mucin fusions linked to Glycosylphosphatidylinositol (GPI)-anchors in tissue regeneration and as tumour immune adjuvants**
Chemokin-Mucin Fusionsproteine mit einer GPI-Ankerdomäne und deren Verwendung als Tumor-Immun-Adjuvanzien oder in der Geweberegeneration
Fusions chimokine-mucine liées aux sites d'encrage de la glucophosphatidyl-inositol et leur utilisation dans la régéneration de tissus et comme adjuvant immuno-tumoral

(43) Date of publication of application: 06.06.2007
(73) Proprietor: Apceth GmbH & Co. KG, 81669 München (DE)
(72) Inventor: Huss, Ralf Prof Dr, 83666 Waakirchen (DE); Nelson, Peter, Prof.Dr, 80336 München (DE); Gröne, Hermann-Josef Prof.Dr., 69118 Heidelberg (DE)
(74) Representative: Grund, Martin

(56) References cited:
- WO-A-03/017944
- US-A1- 2003 138 452
- NOTOHAMIPRODJO MIKE ET AL: "Generation of GPI-linked CCL5 based chemokine receptor antagonists for the suppression of acute vascular damage during allograft transplantation" PROTEIN ENGINEERING DESIGN & SELECTION, vol. 19, no. 1, 26 October 2005 (2005-10-26), pages 27-35, XP002374016 ISSN: 1741-0126
- FLANAGAN K ET AL: "Chemokines and cancer" CANCER INVESTIGATION, MARCEL DEKKER INC, US, vol. 20, no. 5-6, August 2002 (2002-08), pages 825-834, XP009062016 ISSN: 0735-7907
- PREMKUMAR DANIEL R D ET AL: "Properties of exogenously added GPI-anchored proteins following their incorporation into cells" JOURNAL OF CELLULAR BIOCHEMISTRY, vol. 82, no. 2, 26 April 2001 (2001-04-26), pages 234-245, XP002374017 ISSN: 0730-2312
- SHIMAOKA TAKESHI ET AL: "Cell surface-anchored SR-PSOX/CXC chemokine ligand 16 mediates firm adhesion of CXC chemokine receptor 6-expressing cells." JOURNAL OF LEUKOCYTE BIOLOGY, vol. 75, no. 2, February 2004 (2004-02), pages 267-274, XP009063730 ISSN: 0741-5400
- HASKELL CHRISTOPHER A ET AL: "Unique role of the chemokine domain of fractalkine in cell capture: Kinetics of receptor dissociation correlate with cell adhesion" JOURNAL OF BIOLOGICAL CHEMISTRY, vol. 275, no. 44, 3 November 2000 (2000-11-03), pages 34183-34189, XP002374019 ISSN: 0021-9258
- FLANAGAN K ET AL: "Local delivery of recombinant vaccinia virus expressing secondary lymphoid chemokine (SLC) results in a CD4 T-cell dependent antitumor response" VACCINE, BUTTERWORTH SCIENTIFIC. GUILDFORD, GB, vol. 22, no. 21-22, 29 July 2004 (2004-07-29), pages 2894-2903, XP004520428 ISSN: 0264-410X

### SEQUENCE LISTING

<110> LMU München DKFZ Deutsches Krebsforschungszentrum
<120> chemokine-mucin fusions linked to Glycosylphosphatidylinositol (GPI)-anchors in tissue regeneration and as tumor immune adjuvants
<130> 486-1
<160> 1
<170> PatentIn version 3.1
<210> 1
   <211> 1165
   <212> DNA
   <213> Homo sapiens
<400> 1
   gaattcccag tctcagcacc atgaatcaaa ctgcgattct gatttgctgc cttatctttc    60
   tgactctaag tggcattcaa ggagtacctc tctctagaac cgtacgctgt acctgcatca   120
   gcattagtaa tcaacctgtt aatccaaggt ctttagaaaa acttgaaatt attcctgcaa   180
   gccaattttg tccacgtgtt gagatcattg ctacaatgaa aaagaagggt gagaagagat   240
   gtctgaatcc agaatcgaag gccatcaaga atttactgaa agcagttagc aaggaaatgt   300
   ctaaaagatc tcctgctaga ggcggcacct tcgagaagca gatcggcgag gtgaagccca   360
   ggaccacccc tgccgccggg ggaatggacg agtctgtggt cctggagccc gaagccacag   420
   gcgaaagcag tagcctggag ccgactcctt cttcccagga agcacagagg gccctgggga   480
   cctccccaga gctgccgacg ggcgtgactg gttcctcagg gaccaggctc cccccgacgc   540
   caaaggctca ggatggaggg cctgtgggca cggagctttt ccgagtgcct cccgtctcca   600
   ctgccgccac gtggcagagt tctgctcccc accaacctgg gcccagcctc tgggctgagg   660
   caaagacctc tgaggccccg tccacccagg acccctccac ccaggcctcc actgcgtcct   720
   ccccagcccc agaggagaat gctccgtctg aaggccagcg tgtgtggggt cagggacaga   780
   gccccaggcc agagaactct ctggagcggg aggagatggg tcccgtgcca gcgcacacgg   840
   atgccttcca ggactggggg cctggcagca tggcccacgt ctctgtggtc cctgtctcct   900
   cagaagggac ccccagcagg gagccagtgg cttcaggcag ctggacccct aaggctgagg   960
   aacccatcca tgccaccatg gacccccaga ggctgggcgt ccttatcact cctgtccctg  1020
   acgcccaggc tgccacccgg aggcaggcta gaacaacctg tatcccaagc agcggtcatt  1080
   caagacacag atatgcactt atacccatac cattagcagt aattacaaca tgtattgtgc  1140
   tgtatatgaa tgtattatag tcgac                                         1165

## Description

The present invention relates to chemokine-mucin fusions linked to glycosylphosphatidylinositol (GPI)-anchors and their use as anti-cancer adjuvants and as agents in tissue regeneration and suppression of vascular damage. GPI-linked chemokines are incorporated in the surface membrane of tumour cells and effect a recruitment of cytotoxic immune cells to the tumour site following injection *in vivo.* Leukocytes, cytotoxic T-cells and NK cells target the chemokine-GPI-anchored tumour cells and modulate cell-mediated lysis of the tumour cells. The efficiency of GPI-anchoring and modulation of immune cells can be further enhanced by linking the chemokine to a mucin domain followed by the GPI-anchor. The GPI-anchored chemokines, with mucin domain, are remarkably useful for the inhibition of tumour growth, tissue regeneration, and suppression of acute vascular damage to allografts.

### BACKGROUND OF THE INVENTION

The finding of an efficient agent for the inhibition of tumour growth is still one of the most demanding tasks in cancer therapy. The use of immunostimulatory cytokines or chemokines in tumour vaccines has become a promising strategy in cancer immunotherapy (Adam et al., 2003; Coussens and Werb, 2002; Dranoff, 2003; Mackensen et al., 1997b). The aim of this approach is the activation of tumour-specific cytotoxic T-lymphocytes, or the activation of other lymphocytes with anti-tumour potential.

Strategies that provide high levels of immunostimulatory cytokines locally at the site of the tumour have demonstrated some therapeutic efficacy. Two main strategies have been used to achieve expression of cytokines in the tumour: In the ex vivo immunogene therapy approach, autologous cytokine secreting cells are injected in the tumour: In this approach, host cells (tumour cells or fibroblasts) are transfected *ex vivo* with an expression vector carrying a cytokine gene, and re-implanted in the host (Adam et al., 2003; Coussens and Werb, 2002; Dranoff, 2003; Mackensen et al., 1997a; Zarour and Kirkwood, 2003). In the in vivo immunogene therapy approach, the vector carrying the cytokine gene is directly injected at the tumour site (Coussens and Werb, 2002; Dranoff, 2003; Mackensen et al., 1997a; Adam, 2003). A third novel strategy will be detailed in this application, namely, the direct application of a modified chemokine to the tumour or peritumoural environment that will remain fixed at a defined concentration at the site of injection.

### Toll-like receptors in immunity

Toll-like receptors (TLRs) are a family of type I transmembrane proteins that play a crucial role in both innate and adaptive immunity. Toll-like receptors recognize invading pathogens via pattern recognition receptors that bind pathogen-associated molecular patterns, small molecular sequences consistently found on pathogens. Binding of Toll-like receptors leads to activation of the innate immune system, resulting in release of pro-inflammatory cytokines from the monocyte-macrophage system, and the activation and maturation of dendritic cells (Pasare and Medzhitov, 2005). The intratumoural injection of CpG oligonucleotides, a ligand for Toll-like receptor 9, leads to the local production of proinflammatory cytokines such as interleukin-12 and downregulates cytokines, such as interleukin-10, that are essential in mediating the anergic state of effector immune cells (Krieg, 2003). This in turn can help revert the effect of an immune suppressive tumour microenvironment and activate tumour-infiltrating effector cells in situ (Kreig, 2003).

In the recent years it was found that inflammation plays an important role in tumour growth. The tumour microenvironment, which is largely formed by inflammatory cells and their interactions, dictates to a significant degree, the neoplastic process (Murphy, P. M. (2001); Coussens, L. M., and Werb, Z. (2002)). Inappropriate inflammatory cells can help foster tumourogenesis while other leukocytes can help contain tumour growth and spreading (Coussens, L. M., and Werb, Z. (2002); Dranoff, G. (2003); Luznik, L. et al. (2003)). Under which conditions a specific population of leukocytes has a positive or negative effect on the progression of a given type of tumour is not always apparent. The ability to control the selective recruitment and activation of leukocytes within the context of a tumour will provide insight into the relative importance of specific effector leukocytes in defined tumour environments.

### Leukocyte recruitment

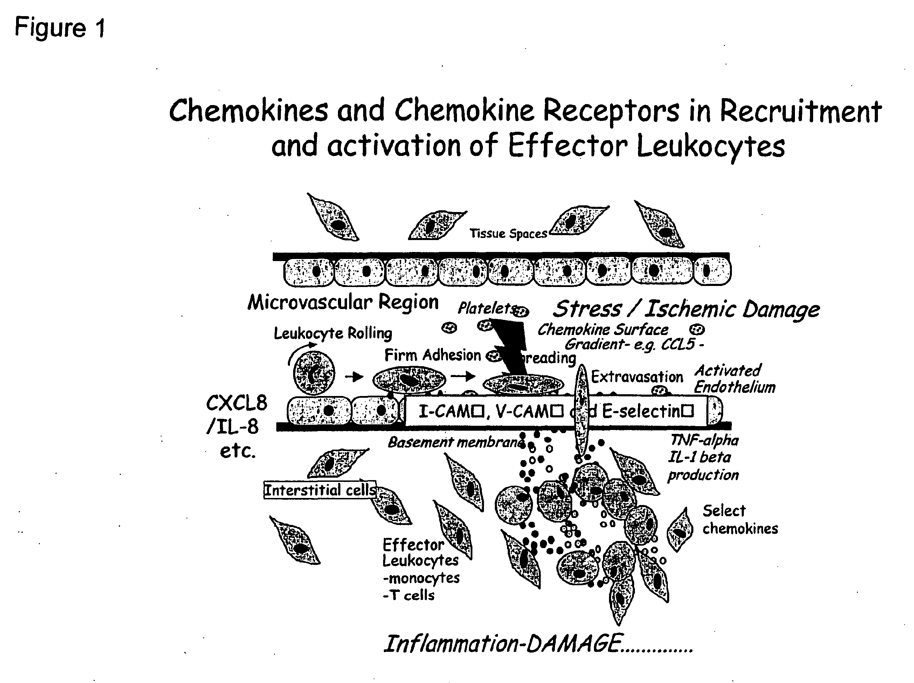

Leukocyte infiltration into tissue involves a series of complex interactions between molecules expressed on the leukocyte, the endothelial surface, and the extracellular matrix (Nelson, P. J., and Krensky, A. M. (2001)). The initial step in leukocyte infiltration entails leukocyte capture via transient interactions between selectins and vascular addressins of leukocytes along the microvascular surface (Nelson, P. J., and Krensky, A. M. (2001); Wong, M. M., and Fish, E. N. (2003) see Figure 1). This selectin/addressin-dependent cell-cell interaction provides the transient, reversible, adhesive interactions between the rolling leukocyte and the endothelial surface required before leukocytes can undergo a firm adhesion to the endothelium. The induction of shear resistant adhesion involves the activation of leukocyte integrins (Nelson, P. J., and Krensky, A. M. (2001)). This process is triggered by the action of chemokines (abbrev. for chemotactic cytokines) bound to the endothelial surface (D'Ambrosio, D. et al. (2003)). The leukocyte then undergoes spreading, diapedesis through junctions between the endothelial cells, and then extravasates by partially digesting and loosening the basement membrane. This allows the leukocyte to enter interstitial spaces.

### Chemokines

Chemokines play an important role in the recruitment and activation of immune cells. The chemokines represent a large family of cytokines that were first characterized by their ability to induce the selective migration of leukocyte subpopulations (Wong, M. M., and Fish, E. N. (2003); Bacon, K. et al. (2002); Murphy, P. M. (2002); Murphy, P. M., et al. (2000)). Chemokines working in concert with selectins and integrins act as the directional cues to sort, direct and fine tune leukocyte trafficking (Nelson, P. J., and Krensky, A. M. (2001)). In addition to the activation of leukocyte-expressed integrins, chemokines can also activate and modulate effector function in leukocytes, influence hematopoesis, and modulate apoptosis and angiogenesis (Nelson, P. J., and Krensky, A. M. (2001); Murphy, P. M. et al. (2000)).

With more than 45 chemokines and 20 chemokine receptors described to date the cytokine family displays a complex biology (Murphy, P. M. et al. (2000); Dong, V. M. et al. (2003)). Chemokines are separated into four branches or subfamilies based upon the positioning of the first two cysteine residues in their primary amino acid sequence: the C, CC, CXC and CX₃C superfamily of cytokines (Murphy, P. M., et al. (2000)).

The CC and CXC subfamilies represent the majority of chemokines. In the CC subfamily the first two cysteine residues lie adjacent to one another. In the CXC family they are separated by a single random amino acid designated as X. A subgroup of CXC chemokines has an additional E-L-R-CXC amino acid motif (glutamic acid-leucine-arginine-cysteine-X-cysteine) (ELR+). The ELR+ CXCL chemokines are angiogenic while the ELR- family members are angiostatic (Bernardini, G. et al. (2003)). The two other branches include the C chemokines and fractalkine/CX₃CL1 the only known CX₃C chemokine. CX₃CL1 is expressed as a transmembrane molecule on the cell surface. In its extracellular domain, the first 76 amino acids of fractalkine/CX3CL1 comprise a chemokine domain followed by an extended mucin-like stalk, a transmembrane domain and an intracellular domain (Haskell, C. A. et al. (2000)). CXCL16 is a CXC chemokine with a structure similar to fractalkine. The membrane-bound forms of fractalkine and CXCL16 can capture leukocytes under physiological flow conditions in an integrin- or G-protein-independent manner (Haskell, C. A. et al. (2000); Chapman, G. A. et al. (2000)). The expression of fractalkine or CXCL16 thus enables bypassing of the first two steps of the adhesion cascade (i.e. rolling and triggering) by mediating cell adhesion between circulating leukocytes and endothelial cells and facilitating extravasation (Ono, S. J. et al. (2003); Lucas, A. D. et al. (2001); Umehara, H. et al. (2001); Nakayama, T. et al. (2003)).

The actions of chemokines are mediated through a family of seven transmembrane serpentine Gi/Go-protein coupled receptors. Each chemokine receptor has a distinct chemokine specificity and a restricted expression on subclasses of leukocytes (D'Ambrosio, D. et al. (2003); Bacon, K. et al. (2002)). The nomenclature and classification used to describe the chemokine receptors is based upon the class of chemokine ligands that interact with each receptor (i.e. C, CC, CXC and CX₃C receptors) (Bacon, K. et al. (2002); Murphy, P. M. (2002)). The differential expression of the receptors by distinct leukocyte subsets is an important component of the specificity of chemokine action (Nelson, P. J., and Krensky, A. M. (2001)).

### Roles of Chemokines in Tumour Biology

Since the infiltration of leukocytes from the peripheral circulation into the parenchyma is essential to immune surveillance, this inflammatory response is an important factor in the initiation and amplification of an anti-tumour immune response. Most of the tumour cells bear antigens that are recognized by the immune system. However, this last line of defense against cancer often fails because of a protected environment. Such a protected environment is responsible that tumour cells are invisible to the immune system. More specifically, the endothelium provides a barrier to immune cells, and in the absence of an inflammatory or "danger" signal, no vigorous immune response is mounted.

The use of immunostimulatory cytokines in tumour vaccines has become a promising strategy in cancer immunotherapy (Coussens, L. M., and Werb, Z. (2002); Mackensen, A. et al. (1997); Adam, J. K. et al. (2003)). The general goal of this approach is the activation of tumour-specific cytotoxic T-lymphocytes (CTL) that are capable of eliminating tumour cells, or the activation of other leukocytes having anti-tumour potential. Strategies that provide high levels of immunostimulatory cytokines locally at the site of the tumour have demonstrated some therapeutic efficacy. However, in some cases cytokines have been shown to increase the tumourigenicity and metastatic properties of tumour cells (Coussens, L. M., and Werb, Z. (2002); Dranoff, G. (2003)). Because of their unique biologic actions, some chemokines have been found to effect tumour biology.

It is therefore an object of the present invention to provide an efficient anti-tumour agent on the basis of chemokines, wherein the agent is capable of inhibiting tumour growth both *in vivo* and *in vitro*. The invention is based on the surprising finding that glycosylphosphatidylinositol (GPI)-anchored chemokines, with or without a mucin domain, elicit an immune response against the tumour cells. The immune response is characterized by a recruitment and activation of leukocytes, NK cells and cytotoxic lymphoytes (CTL). The inventors found that chemokines that are anchored by GPIs are incorporated into the cell surface membranes and retain native protein function when purified and added to the cells.

### Role of Chemokines in Vascular Damage of Allografts

Apart from their potential role in tumour biology, chemokines are involved in immune rejection of solid organ transplants, where the direct recruitment of effector leukocytes from the peripheral circulation into interstitial tissues is controlled in part by the actions of chemokines (Haskell et al., 2002; Nelson and Krensky, 2001). Chemokines that are sequestered in solid phase on the endothelium (immobilized via electrostatic interactions) act as signposts for directing the selective recruitment of circulating leukocytes (Murphy, 2002; Weber et al., 2001; Wells et al., 1999). In response to chemokine stimulation, leukocytes upregulate integrins and undergo a firm adhesion to the endothelial surface (Murphy, 2002; Weber et al., 2001). The leukocyte then migrates through the endothelium and enters the tissue space. Blocking or modifying this process is considered to be an important step in the control of transplant rejection.

One chemokine that was found to be a chemotactic agent for "memory" CD4+ T cells, monocytes and eosinophils is RANTES/CCL5. This chemokine is produced by many different cell types during allograft rejection (Nelson and Krensky, 2001; Nelson et al., 1997; Schall et al., 1990). CCL5 acts through the receptors CCR1, CCR3 and CCR5 (Murphy, 2002). Human RANTES/CCL5 is bioactive in mouse and rat (Stojanovic et al., 2002; von Hundelshausen et al., 2001; Wiedermann et al., 1993). Blocking analogues of the human CCL5 protein bind to human and rodent CCL5 receptors with high avidity but do not induce signaling (Proudfoot et al., 1999; Proudfoot et al., 1996).

One problem associated with the use of recombinant proteins, however, is that a systemic administration of recombinant proteins can be quite expensive. In order to demonstrate a biological effect in transplantation experiments, the RANTES based antagonistic protein had to be administered daily over an extended period of time (Grone et al., 1999; Song et al., 2002). This could be problematic as high circulating protein levels could have unwanted side effects on other organ systems.

It is a therefore a further object of the present invention to reduce the above-mentioned side effects in allografts by selectively applying a CCL5 based antagonistic protein to the vascular cell surface prior to transplantation.

### SUMMARY OF THE INVENTION

The invention relates to the constructs, nucleic acid molecules, plasmid, host cell, vector compositions, use, and method as defined in the appended claims. Findings that a chemokine linked to a glycosylphosphatidylinositol (GPI) anchor results (1) in a modulation and recruitment of immune cells and immune-mediated killing of tumour cells, (2) tissue regeneration and (3) a reduction of immune-induced vascular damage in allograft transplantation are disclosed. Chemokines that are anchored by GPIs, when purified and added to cells, are incorporated into their cell surface membranes and retain their full native protein function.

The present inventors have devised a method that enhances a selective immune response by initiating the direct recruitment and activation of specific subsets of immune effector cells into the tumour environment and by this action augment the selective immune response against the tumour.

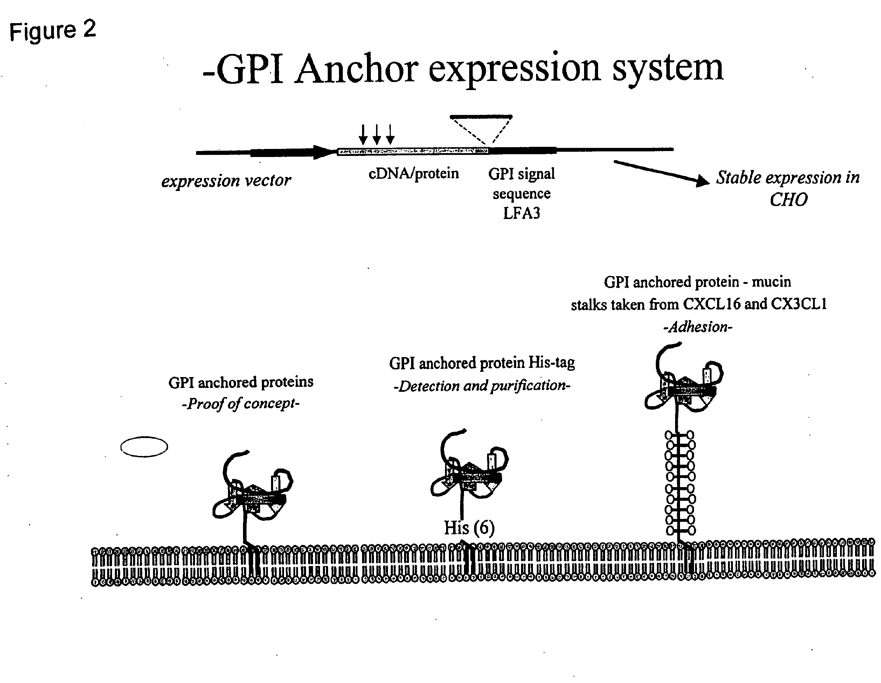

It was one objective to utilize the biological activity of chemokines and their receptors to enhance inflammation in tumour environments. By substituting a GPI signal domain coding sequence for the carboxyl terminal region of a chemokine, fusion constructs were generated that encode a series of GPI-linked chemokine proteins (see Figure 2). One or more mucin adhesion domains have been additionally incorporated. The incorporation of one or more mucin domains or stalks between the chemokine and the GPI-anchor was found to significantly increase the incorporation rate in the outer membrane of cells. As a consequence, this caused an enhanced leukocyte adhesion, and, thus, more efficient killing of tumour cells. The mucin domains as used in the present invention are largely composed of serine/threonine/glycine/proline residues shown to facilitate cell-cell interactions.

In a preferred embodiment, the chemokine fusion construct comprises an amino acid sequence of a native chemokine or a biologically active fragment thereof, wherein said chemokine or biologically active fragment thereof is linked to a mucin domain followed by a glycosylphosphatidylinositol (GPI)-anchor. In one embodiment, the resultant fusion construct is expressed as recombinant protein. The recombinant expression fusion construct can be purified and added to the tissue to be treated.

In a preferred embodiment, the above-mentioned mucin domain is a membrane-bound mucin domain, and preferably comprises an amino acid sequence encoded by a gene selected from the group consisting of MUC1, MUC3A, MUC3B, MUC4, MUC11, MUC12, MUC16, and MUC17, or a portion thereof.

In a further preferred embodiment the mucin domain comprises the mucin-stalk of the surface-associated chemokine CXCL16 or fractalkine (CX3CL1).

As used in the present invention, the chemokine is a member of the C, CC, CXC or CX₃C subfamily of chemokines. Preferably, the C subfamily chemokine is selected from the group consisting of XCL1/Lymphotactin/SCM-1a, and XCL2/SCM-1β; the CC subfamily chemokine is selected from the group consisting of CCL1/I-309, CCL2/MCP-1, CCL3/MIP-1α, CCL3L1/LD78β, CCL4/MIP-1β, CCL5/RANTES, CCL6, CCL7/MCP3, CCL8/MCP-2, CCL9/CCL10, CCL11/Eotaxin, CCL12, CCL13/MCP-4, CCL14/HCC-1, CCL15/HCC-2/Lkn-1/MIP-1δ, CCL16/HCC-4/LEC/LCC-1, CCL17/TARC, CCL18/DC-CK1, CCL19/MIP-3β/ELC, CCL20/MIP-3α/LARC, CCL21/6Ckine/SLC, CCL22/MDC, CCL23/MPIF-1/CKb8, CCL24/Eotaxin-2, CCL25/TECK, CCL26/Eotaxin-3, CCL27/CTACK, CCL28/MEC; and the CXC subfamily chemokine is selected from the group consisting of CXCL1/GROα, CXCL2/GROβ, CXCL3/GROγ, CXCL4/PF4, CXCL5/ENA-78, CXCL6/GCP-2, CXCL7/NAP-2, CXCL8/IL-8, CXL9/Mig, CXCL10/IP-10 CXCL11/I-TAC, CXCL12/SDF-1α/β, CXCL13BCA-1, CXCL14/BRAK, CXCL15, CXCL16

In a preferred embodiment, the chemokine is selected from the group consisting of fractalkine/CX₃CL1, CXCL16, IL-8/CXCL8, IL-10, IP-10/CXCL10, RANTES/CCL5.

Preferably, the fusion construct contains one or more GPI signal sequences to direct GPI-anchoring, and the GPI-anchor is preferably derived from the lymphocyte function-associated antigen (LFA-3), or a portion thereof.

The addition of the fusion construct of the invention to cells results in an incorporation of the chemokine into the cell membranes of tumour cells by means of its GPI anchor.

In a further aspect, the invention relates to a nucleic acid molecule, which comprises a nucleic acid sequence that encodes for a fusion construct of the invention. In a further aspect, the invention relates to an expression plasmid which comprises such a nucleic acid molecule and additional expression elements. The expression plasmid or a vector derived thereof can be used for recombinant expression of the proteins of the invention in suitable eukaryotic (e.g. human, mice or hamster cell lines) or prokaryotic host cells (e.g. Escherichia coli).

In a further aspect, the invention relates to a pharmaceutical composition, which comprises the fusion construct or the nucleic acid molecule of the invention, and a pharmaceutically acceptable carrier.

The fusion constructs of the invention (chemokine-mucine-GPI) can be used for the treatment of cancer, e.g. breast cancer, renal cancer, prostate cancer, seminomas, melanomas, teratomas, neuroblastomas, gliomas, rectal cancer, endometrial cancer, kidney cancer, adrenal cancer, thyroid cancer, blood cancer, skin cancer, cancer of the brain, cervical cancer, intestinal cancer, liver cancer, colon cancer, stomach cancer, intestine cancer, gastrointestinal cancer, lymph node cancer, esophagus cancer, colorectal cancer, pancreas cancer, ear, nose and throat (ENT) cancer, cancer of the uterus, ovarian cancer and lung cancer.

The fusion construct can be locally administered as anti-tumour adjuvant. Furthermore, the fusion construct is useful for the treatment of residual cancer after surgery of a primary tumour, e.g. in breast cancer patients and patients with glioblastoma (astrocytoma VI). Preferably, the fusion construct is administered at a concentration of 0,5 to 5 µg/ml, preferably 1 µg/ml. In one embodiment, the fusion construct is administered by spraying into the wound and/or injection into regions that are not available for surgery.

In addition to their utility in tumour regression, the chemokine-mucin-GPI fusion constructs of the invention can be used for tissue regeneration and suppression of acute vascular damage during allograft transplantation.

Two main strategies have been used to achieve expression of cytokines in the tumour: In the *ex vivo* immuno-gene therapy approach, autologous cytokine secreting cells are injected in the tumour, and subject cells (tumour cells or fibroblasts) are harvested, transfected *ex vivo* with an expression vector carrying a cytokine gene, and reimplanted in the subject. In the *in vivo* immunogene therapy approach, the vector carrying the cytokine gene is directly injected at the tumour site. In a third strategy, the direct addition of a modified chemokine adjuvant will remain fixed at a defined concentration to the tumour site.

In a further aspect the invention relates to an *in vitro* method for inhibition of cancer cell proliferation comprising the step of subjecting a cancer cell line to an effective amount of chemokine-mucin-GPI fusion construct.

### DEFINITIONS

With the term "GPI" as used herein is meant glycoinositol phospholipids, in particular, glycosylphosphatidlyinositol as described in Medof et al., 1996. These phospholipid-like anchors have a common structure for membrane attachment irrespective of protein function. GPI anchoring units are composed of a linear glycan containing a phosphoethanolamine, three mannose residues, and a nonacetylated glucosamine linked to an inositol phospholipid. The GPI sequence contains the signals that direct GPI anchoring.

With the term "mucin" or "mucin domain" as used herein is meant a membrane bound or non-membrane glycoprotein component. Usually, membrane-bound mucins exhibit hydrophobic sequences or transmembrane domains responsible for their anchoring in the lipid bilayer and, optionally, contain one or several von Willebrandt factor-like domains, which function in the oligomerization of mucin monomers and in the packaging into secretory vesicles. The term "mucin" or "mucin domain" as used herein also encompasses mucin-stalks or mucin-like domains such as the mucin-stalks found in the chemokines CXCL16 or fractalkine (CX3CL1).

A "chemokine-GPI" fusion construct as used herein relates to a chemokine that is fused directly to a GPI-linkage sequence (see Figure 2). The chemokine-GPI fusion construct is designed by substituting the 3'-mRNA or cDNA end sequence of naturally GPI-anchored proteins (i.e., a sequence that contains the signals that direct GPI anchoring) for the endogenous chemokine 3'-mRNA or cDNA end sequence.

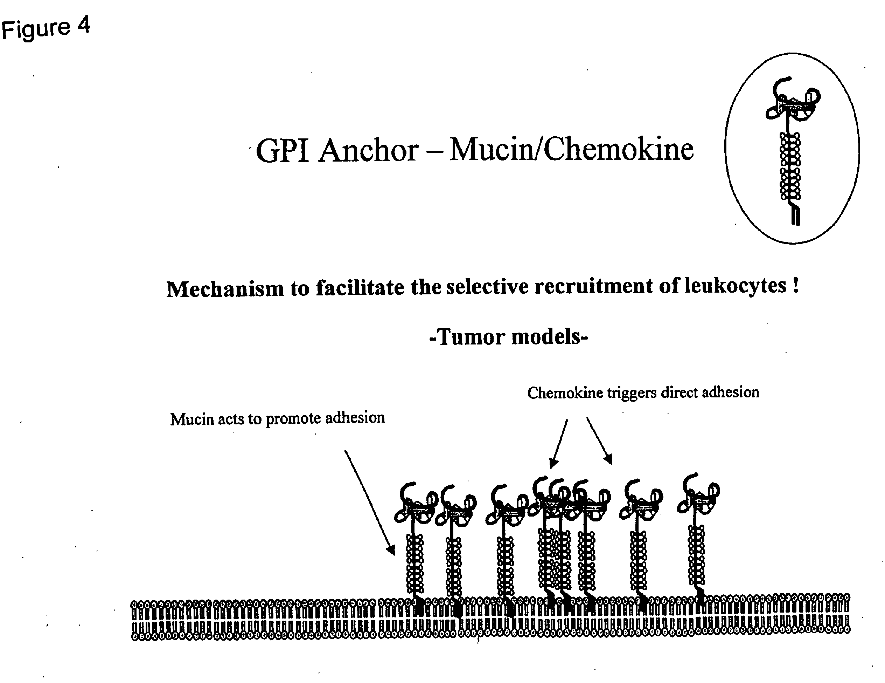

A "chemokine-mucin-GPI" as used herein relates to a chemokine that is directly fused to a mucin domain followed by a GPI-linking sequence (see Figure 4). The chemokine-mucin-GPI fusion construct is designed as described for chemokine-GPI but further comprises the amino acid sequence of a mucin domain or stalk between the amino acid sequences of the chemokine and GPI.

### DETAILED DESCRIPTION

Chemokines play central roles in the selective attraction, migration and activation of defined subsets of immune effector cells. The present invention takes advantage of the direct recruitment of immune effector leukocytes from the peripheral circulation and the subsequent control of effector function.

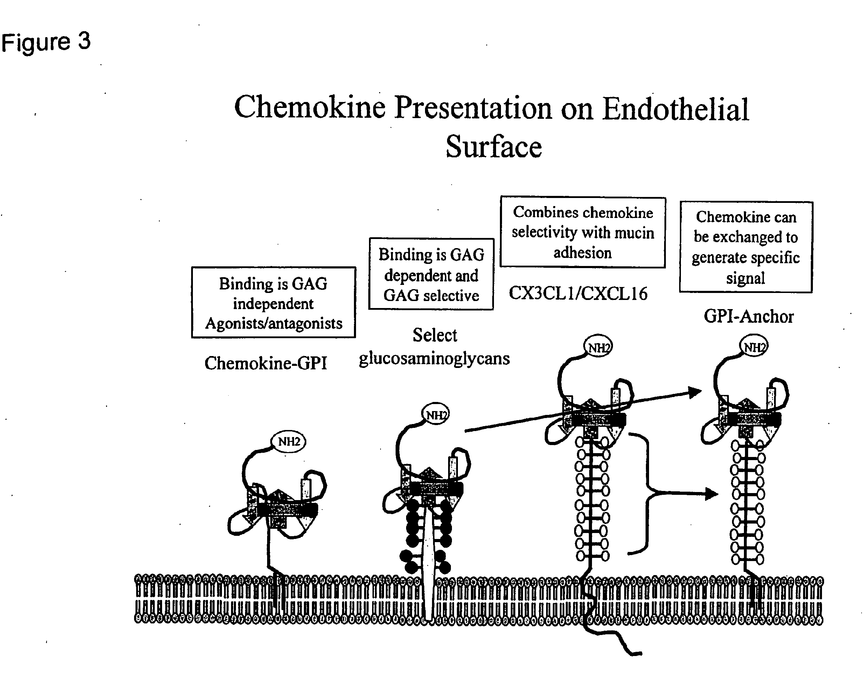

For this purpose, the structural and functional determinants of chemokines have been combined with a GPI anchor and various linkers to generate a series of highly flexible immunomodulatory reagents (see Figure 2). Chemokines anchored by glycoinositol phospholipids (GPIs), when purified and added to cells *in vitro* or tissues *in vivo,* are incorporated into surface membranes of cells (see Figure 3). The GPI-linked chemokines are incorporated in the membranes of tumour cells resulting in a recruitment of immune effector leukocytes to the tumour site and a subsequent immune cell-mediated cytotoxic killing of the tumour cells. The ability to recruite immune cells to mediate killing of tumour cells makes the fusion constructs particularly attractive for tumour therapy.

### GPI-anchoring of Chemokines

The approach of anchoring chemokines with GPI offers multiple advantages over traditional gene transfer approaches known in the prior art. For instance, the method of the invention is applicable to cells that are hard or impossible to transfect (e.g. primary microvascular endothelium, primary target cells, etc). Furthermore, the amount of protein added to the cell surface can be controlled and quantitated (by FACS or immunoflourescence). Furthermore, through molecular engineering it is possible to express an additional epitope tag that assists in protein purification as well as monitoring of reagent during experiments. The agent can be injected directly into the tumour or peritumourial area and the effect of selective leukocyte recruitment on tumour growth determined.

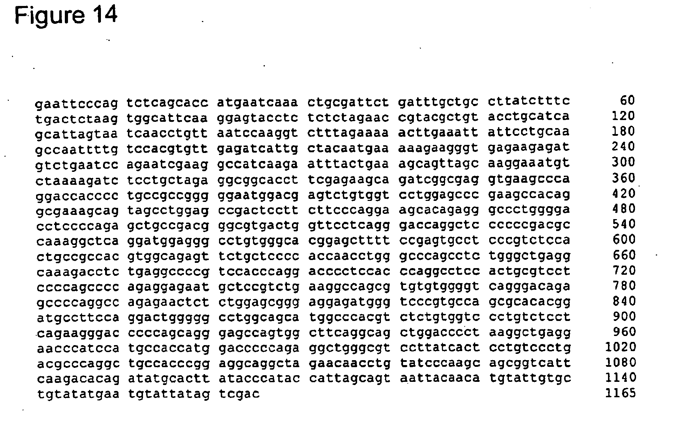

The fusion constructs of the invention are prepared by substituting the mRNA derived cDNA sequence from a GPI linked chemokine protein that contains the GPI signal domain for the carboxyl terminal region of a protein of interest. For the construction of the fusion constructs of the invention, i.e. chemokine-mucin-GPI, either the full-length sequence of the chemokine can be used or a functionally active portion thereof, which retains the activity of the chemokine. Likewise also a portion of the GPI sequence can be used instead of the full GPI-anchor, as long as the portion allows for the association of the chemokine protein with the surface cell membrane on cancer cells. An example of such a construct is shown in Figure 14, exemplifying the nucleic acid sequence of IP-10-fractalkine-GPI.

The chemokine-mucin-GPI nucleic acid fusion construct of the invention can then be expressed in any suitable cell line or host cell to obtain a functional recombinant chemokine protein. In a preferred embodiment the cell line used for expression of or chemokine-mucin-GPI is an eukaryotic (e.g. vertebrate) cell line (e.g. human, mice, hamster) or a prokaryotic cell line (e.g. Escherichia coli). One preferred cell line to express the fusion constructs of the invention is the Chinese Hamster Ovary (CHO) cell line. More preferably, the polypeptide fusion constructs are derived as recombinantly expressed proteins from a suitable human cell line.

For the recombinant expression of the GPI-anchored chemokine proteins of the invention any of the suitable known vectors or plasmids can be used as long as they result in the desired protein, retaining the function of the chemokine.

GPI-linked chemokines that can be used in the present invention comprise the C, CC, CXC or CX₃C subfamily of chemokines. Preferably, the C subfamily chemokine is selected from the group consisting of XCL1/Lymphotactin/SCM-1a, and XCL2/SCM-1β; the CC subfamily chemokine is selected from the group consisting of CCL1/I-309, CCL2/MCP-1, CCL3/MIP-1α, CCL3L1/LD78β, CCL4/MIP-1β, CCL5/RANTES, CCL6, CCL7/MCP3, CCL8/MCP-2, CCL9/CCL10, CCL11/Eotaxin, CCL12, CCL13/MCP-4, CCL14/HCC-1, CCL15/HCC-2/Lkn-1/MIP-1δ, CCL16/HCC-4/LEC/LCC-1, CCL17/TARC, CCL18/DC-CK1, CCL19/MIP-3β/ELC, CCL20/MIP-3α/LARC, CCL21/6Ckine/SLC, CCL22/MDC, CCL23/MPIF-1/CKb8, CCL24/Eotaxin-2, CCL25/TECK, CCL26/Eotaxin-3, CCL27/CTACK, CCL28/MEC; and the CXC subfamily chemokine is selected from the group consisting of CXCL1/GROα, CXCL2/GROβ, CXCL3/GROγ, CXCL4/PF4, CXCL5/ENA-78, CXCL6/GCP-2, CXCL7/NAP-2, CXCL8/IL-8, CXCL9/Mig, CXCL10/IP-10, CXCL11/I-TAC, CXCL12/SDF-1α/β, CXCL13/BCA-1, CXCL14/BRAK, CXCL15, CXCL16

In a preferred embodiment, the chemokine is selected from the group consisting of fractalkine/CX₃CL1, CXCL16, IL-8/CXCL8, IL-10, IP-10/CXCL10, RANTES/CCL5.

In a preferred embodiment of the invention, the chemokine is directly linked to the GPI-anchor. Unlike conventional polypeptide anchors, which have different transmembrane sequences and connect to specific cytoplasmic extensions, these phospholipid-like anchors use a common structure as a general mechanism for membrane attachment irrespective of protein function. GPI anchoring units are composed of a linear glycan containing a phosphoethanolamine, three mannose residues, and a non-acetylated glucosamine linked to an inositol phospholipid. They are prefabricated in the endoplasmic reticulum (ER) and are added to primary translation products at the time of their translocation across the ER membrane. The GPI-modified products then are glycosylated in the ER and Golgi, and subsequently transported to the cell surface.

Preferred GPI-linking sequences that can be used in the present invention are derived from GPI-anchors that are isolated from, for example, enzymes such as alkaline phosphatase, acetylcholinesterase, 5' nucleotidase (CO73); adhesion molecules such as lymphocyte function-associated antigen (LFA-3; CD58), neural cell adhesion molecule (NCAM); complement regulatory proteins such as decay accelerating factor (DAF or CD55), or others such as the Fey receptor type III B (Fc-γ-RIII or CD16b), Thy-1 (CD90), Qa-2, Ly-6A, Membrane inhibitor of reactive lysis (MIRL or CD59). For the purpose of the present invention, the lymphocyte function-associated antigen (LFA-3) is preferred. It is preferred that the fusion construct contains one or more GPI signal sequences. These signal sequences have the function to direct anchoring of GPI in the cell surface membrane of tumour cells. The skilled person will recognize that also any other of the known GPI-anchors can be used for the practice of the present invention.

### Mucin domains enhance leukocyte infiltration

The fusion construct contains one or more mucin domains that can be arranged between the chemokine and the GPI-anchor (see Figure 4). It was surprisingly found that the incorporation of one ore more mucin domains, in addition to the chemokine and GPI, resulted in an enhanced leukocyte recruitment, when added to cells. The mucin domains can be arranged concurrently to each other. Mucins are membrane-bound or non-membrane glycoprotein components that were first identified in secreted mucus lining the surfaces of glandular epithelia. Membrane-bound mucins exhibit hydrophobic sequences or transmembrane domains responsible for their anchoring in the lipid bilayer.

At present, a total of 21 genes have received the appellation MUC: MUC1-2, MUC3A, MUC3B, MUC4, MUC5AC, MUC5B, MUC6-13, MUC15-20 (Moniaux N, et al., 2004). The five common features of a mucin are: (1) secretion into the mucus layer, (2) high molecular weight O-glycoprotein, (3) presence of a tandem repeat array encoded by a unique and centrally positioned large exon, (4) presence of a predicted peptide domain containing a high percentage of serine and threonine residues, and (5) a complex pattern of mRNA expression. With one exception (MUC7), the secretory mucins (MUC2, MUC5AC, MUC5B and MUC6) possess one or several von Willebrandt factor-like domains, cysteine-rich peptides, which function in the oligomerization of mucin monomers and in the packaging into secretory vesicles. Typically, secreted mucins are expressed exclusively by specialised epithelial cells, are secreted in the mucus, and display a restricted expression pattern within the human body. The four secretory mucins, also referred to as the gel-forming mucins, have a common architecture with a high level of similarity to the pro-von Willebrand factor. They are also known to harbour five D domains because of their homology to the D domains of the von Willebrand factor.

The membrane-bound mucins are composed of MUC1, MUC3A, MUC3B, MUC4, MUC11, MUC12, MUC16, and MUC17. Membrane-anchored mucins contain a SEA (Sea urchin sperm protein, Enterokinase and Agrin) module, with the exception of MUC4. MUC3A-B, MUC4, MUC11-12, and MUC17 contain two to three epidermal growth factor (EGF)-like domains. Examples of membrane-bound mucins that can be used in the present invention are MUC1, MUC3, MUC4, and MUC12. In a preferred embodiment of the invention, the mucin-stalk of the surface-associated chemokine CXCL16 or fractalkine (CX3CL1) is used. CXCL16 is a member of the CXC chemokine subfamily. Unlike other members of this subgroup, CXCL16 is structurally different and has four distinct domains: a chemokine domain tethered to the cell surface via a mucin-like stalk, which in turn is attached to transmembrane and cytoplasmic domains. Fractalkine (CX3CL1) has a similar structure to that of CXCL16, and both CXCL16 and fractalkine act as adhesion molecules when expressed on cell surface, and upon cleavage from cell surface, the soluble chemokines act as chemoattractants.

The mucin as used in the present invention is preferably a membrane-bound mucin domain and comprises preferably an amino acid sequence of a mucin selected from the group consisting of MUC1, MUC3A, MUC3B, MUC4, MUC11, MUC12, MUC16, and MUC17, or a variant or portion thereof (the above mucins are reviewed in Moniaux N et al., 2004). In another preferred embodiment, a mucin-like stalk is used that is preferably isolated from the surface-associated chemokine CXCL16 or fractalkine (CX3CL1). The skilled person will recognize that any other mucin-like stalks or mucin domains are suitable for use in the present invention.

Preferably, the mucin domain is fused between the 3'-end of the sequence of the chemokine and the 5'end of the GPI anchor sequence by any of the known conventional genetic engineering methods. The resultant chemokine-mucin-GPI fusion constructs of the invention can then be transfected and expressed in any suitable known cell line or host cell. In one embodiment, the resultant constructs are transfected into dihydrofolate reductase (DHFR)-deficient Chinese Hamster Ovary (CHO) cells and selection performed as described (Mack, et al., P.N.A.S. USA 92:7021-7025, 1995). In order to increase the expression rate by gene amplification the transfectants can be exposed to methotrexate.

### Construction of GPI-linked chemokine Fusion Constructs

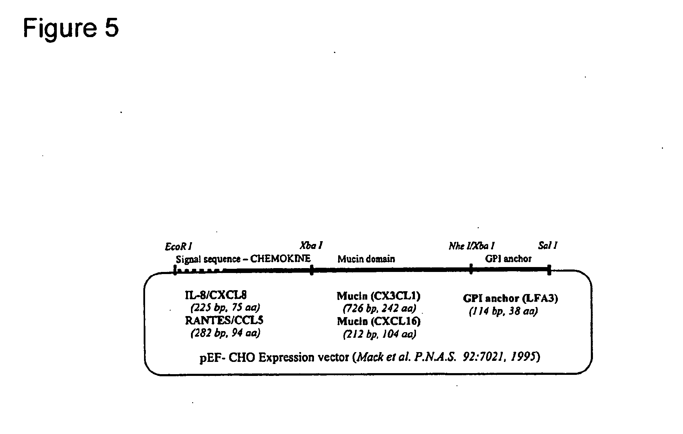

To demonstrate the utility of the GPI-linked chemokines in cancer therapy, tissue regeneration and suppression of acute vascular damage to allografts, a series of constructs have been generated and stably introduced into CHO cells (the constructs are summarized in Table 1). By way of reference examples, Interleukin-8 (IL-8)/CXCL8, IP-10 (interferon-alpha inducible protein 10)/CXCL10 and RANTES/CCL5 have been used to demonstrate the efficiacy of GPI-linked chemokines in tumour and microvascular cell models (see Figure 5). The sequenced nucleic acid sequence of the IP-10-fractalkine-GPI construct is exemplary shown in Figure 14.

Interleukin-8 (IL-8)/CXCL8 enhances inflammation by attracting neutrophils, basophils, and T-cells through CXCR1 and CXCR2). In addition, IL-8/CXCL8 is directly involved in neutrophil activation. In tumour models, expression of the human IL-8 gene in Chinese hamster ovary (CHO) cells dramatically inhibited growth *in vivo* when injected into nude mice. Histological studies revealed neutrophilic infiltration at the site of injection, suggesting a mechanism of action involving site-directed recruitment of neutrophilic granulocytes.

IP-10 (interferon-alpha inducible protein 10)/CXCL10 exerts chemotactic activity through CXCR3 which is expressed preferentially on T helper 1 cells, NK cells and some monocytes. Gene transfer of IP-10 into tumour cells has been found to reduce their tumourogenicity and elicits long-term protective immunity. The angiostatic activity of IP-10/CXCL10 was also shown to mediate tumour regression: Tumour cells expressing IP-10/CXCL10 became necrotic *in vivo.* IP-10/CXCL10 was also shown to mediate the angiostatic effects of IL-12 that lead to tumour regression it also inhibits neovascularization by suppressing endothelial cell differentiation. The sequenced nucleic acid sequence of the IP-10-fractalkine-GPI construct is shown in Figure 14.

RANTES (= Regulated on Activation Normal T cell Expressed, Secreted)/CCL5 acts through three receptors CCR1, CCR5 and CCR3. Its chemoattractive activity is directed toward monocytes, dendritic, T and NK cells, eosinophils and basophils. It can also activate effector function in specific leukocyte subsets including monocytes, T cells and natural killer cells. RANTES/CCL5 is also a potent eosinophil-specific activator of oxidative metabolism and eosinophilic cationic protein release. Human RANTES/CCL5 exerts an antitumoural effect: tumour cells transfected with RANTES/CCL5 gene show a reduced ability to form tumours *in vivo* and elicit an anti-tumour immune response which protects animals from challenge with the parent tumour cells.

In one embodiment, the chemokine RANTES/CCL5 has been linked to GPI with additional mucin stalks. Also included in the invention is the generation of wild type RANTES/CCL5 protein, Met-RANTES (a functional antagonist), a series of mutations that moderate the aggregation of the protein, a mutation in the GAG binding domain of the protein, and finally, various versions of the RANTES/CCL5 protein fixed on the end of mucin stalks taken from CXCL16 or fractalkine/CX3CL1 (Table 1). In other embodiments, GPI-mucin based proteins have been generated based on CXCL10/IP10 and IL-8/CXCL8 as examples.

**Table 1: Examples and reference examples of chemokine-GPI constructs of the invention**

| | |
|---|---|
| **hRANTES/CCL5:** This CC chemokine binds to the receptors CCR1, CCR3 and CCR5. These receptors are expressed on monocytes (CCR1 and CCR5), T cells (CCR1, CCR3 and CCR5), natural killer cells (CCR1 and CCR5) and eosinophils (CCR1 and CCR3). (reference example) | **hRANTES/CCL5-GPI:** Wild type human protein, tends to form large aggregates and binds to heparin. (reference example) |
| **hMetRANTES-GPI:** Contains an addition of a single Met group to amino terminus - acts as a functional antagonist for CCR1, CCR3 and CCR5. The construct is included as a negative control for the experiments detailed here. (reference example) | **hRANTES E26S-GPI:** Single amino acid substitution at amino acid 26 (glutamic acid for serine) renders the resultant RANTES protein to a tetramer. (reference example) |
| **hRANTES E66S-GPI:** Single amino acid substitution at amino acid 66 (glutamic acid for serine) renders the resultant RANTES protein to a dimer. (reference example) | **hRANTES 44AANA47-GPI:** Three alanine residues have been substituted for amino acids found in the wild type protein. This substitution mutant does not bind to heparin - when expressed as a soluble protein, it will no longer signal through CCR1. (reference example) |
| **hRANTES 44AANA47-E26S-GPI:** Non-heparin binding tetramer. (reference example) | **hRANTES 44AANA47-E66S-GPI:** Non-heparin binding dimer. (reference example) |
| **hRANTES-mucin1-GPI:** This expression cassette has incorporated the mucin domain isolated from the surface associated chemokine CXCL16. The mucin domain has been placed between the GPI anchor and the carboxyl end of the chemokine which mimics the structure of CXCL16. The protein has been tested for enhanced leukocyte adhesion. | **hRANTES-mucin2-GPI:** A second construct incorporated the longer and potentially more robust mucin domain isolated from the fractalkine/CX3CL1 chemokine. The fusion protein will be tested for increased leukocyte adhesion. |
| **hIL-8/CXCL8:** The protein acts on CXCR1 and CXCR2 in humans. These receptors are highly expressed on neutrophils and granulocytes. (reference example) | **hIL-8-GPI:** This construct will be used to study the direct effect of IL-8 (reference example) (reference example) |
| **hIL-8-mucin2-GPI:** The mucin domain isolated from the fractalkine/CX3CL1 chemokine has been added to increase leukocyte adhesion. | **hIP-10/CXCL10:** The protein acts on CXCR3 in humans. The receptor is expressed on T cells natural killer cells and some monocytes. (reference example) |
| **hIL-8-GPI:** This construct has been used to study the direct effect of IL-8 (reference example) | **hIL-8-mucin2-GPI:** The mucin domain isolated from the fractalkine/CX3CL1 chemokine has been added to increase leukocyte adhesion. |

| | |
|---|---|
| mucin1 = mucin-stalk of CX3CL1; mucin2 = mucin-stalk of CXCL16 | |

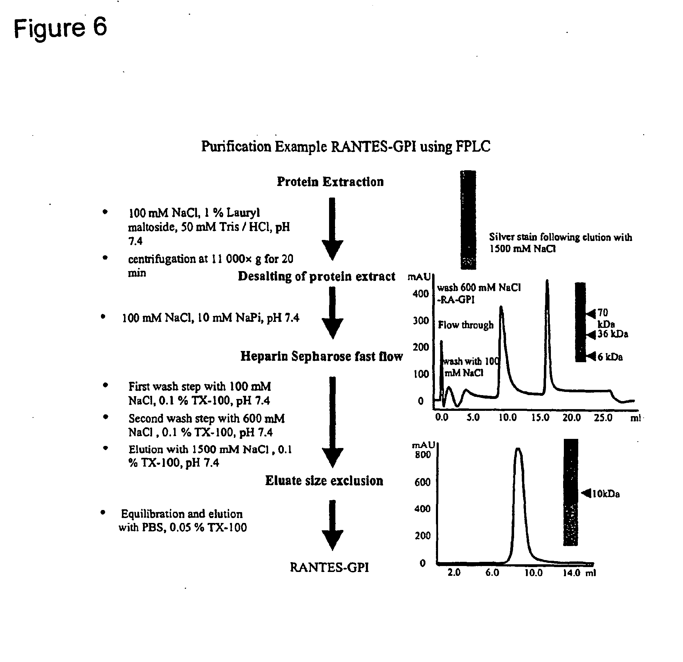

For the purification of the fusion constructs "Fast Pressure Liquid Chromatography" (FPLC) has been applied. The GPI-chemokine products are isolated from the transfected cells, e.g. CHO transfectants, by detergent extraction of the transformed cells and purification on FPLC using a series of chemistries including; heparin sepharose, size exclusion chromatography, DEAE ion exchange resins (see Figure 6).

As an example, GPI-linked chemokines based on the biology of RANTES/CCL5 and IL-10/CXCL10 were used to devise the exemplary purification protocols (see Figure 6). The procedure yields approximately 100 micrograms of protein product when isolated from culture flasks of CHO cells. The so purified proteins have been tested for their efficiency in reincorporation into control cells.

The vector used for expression of RANTES/CCL5 and IL-10/CXCL10 contains a promoter for human elongation factor 1 alpha followed by a multiple cloning site and an internal ribosomal binding site which allows the bicistronic expression of the construct and dihydrofolate reductase (DHFR) used as a selection marker (Mack, et al., P.N.A.S. USA 92:7021, 1995). In one embodiment, the 3' end (carboxyl terminal) of the chemokine protein is fused either directly to a GPI-linkage sequence (e.g. derived from lymphocyte function-associated antigen-3 (LFA-3)) or a mucin domain into the transfection vector. In a preferred embodiment the mucin domain is isolated from CXCL16 or fractalkine (CX3CL1) followed by the GPI signal.

In order to evaluate the efficiency of chemokine-GPI-anchoring in the cell surface membrane, Flourescence Activated Cell Sorting (FACS) analysis using either chemokine or specific monoclonal antibodies can be used to identify stable transfectants producing the highest amount of GPI-anchored protein. FACS analysis following PI-PCL (phosphatidylinositol-specific phospholipase) digestion confirms a successful GPI anchorage.

### Leukocyte Recruitment in vitro and in vivo

The present invention addresses the role of chemokines in the direct recruitment of leukocytes from the periperal circulation. Experiments were performed which show that circulating platelets and the chemokine RANTES/CCL5, contribute to the activation and interaction of leukocytes and endothelium. Binding of RANTES/CCL5 to human endothelial cells can be detected by ELISA or immunofluorescence after perfusion with platelets or exposure to their supernatants. A monocyte and T cell arrest on endothelial monolayers or surface-adherent platelets can be studied with a parallel-wall flow chamber and video microscopy. As a result, it was found that RANTES/CCL5 secreted by thrombin-stimulated platelets is immobilized on the surface of inflamed microvascular or aortic endothelium and triggers shear-resistant monocyte arrest under flow conditions. Moreover, a deposition of RANTES/CCL5 and its effects requires endothelial activation.

As shown in the following examples, the selective chemokine signalling by a GPI-anchored chemokine can recruit monocytes, Th1- and Th2-like T cells. The roles of the RANTES/CCL5 receptors CCR1 and CCR5 on the selective recruitment of monocytes, T(H)1-like T-cell clones, and peripheral T cells enriched for CD45RO(+) "memory" cells were tested in a system in which arrest under flow conditions was triggered by RANTES/CCL5 immobilized to activated endothelium. By using selective nonpeptide receptor antagonists or blocking antibodies, it was found that the RANTES-induced arrest of these cells was mediated predominantly by CCR1. In contrast, CCR5 mainly contributed to leukocyte spreading in shear flow, and both CCR1 and CCR5 supported transendothelial chemotaxis toward RANTES/CCL5. Thus, further evidence of specialized roles of apparently redundant receptors in distinct steps of leukocyte trafficking is disclosed.

Furthermore, the viral CC chemokine macrophage inhibitory protein-II (vMIP-II) encoded by human herpes virus 8 binds to multiple chemokine receptors. vMIP-II blocks the firm arrest and transmigration of monocytes or T(H)1-like T lymphocytes triggered by RANTES/CCL5 immobilized on activated human microvascular endothelium under flow conditions. In contrast, vMIP-II triggered the firm arrest of eosinophils and T(H)2-like T cells by engaging CCR3. Immunohistochemical analysis of HHV-8-associated Kaposi's sarcoma lesions marked by vMIP-II expression and mononuclear cell infiltration revealed a predominance of T(H)2-type CCR3(+) lymphocytes over T(H)1-type CXCR3(+)/CCR5(+) leukocytes, indicating that as a CCR3 agonist vMIP-II can drive a T(H)2-type immune response *in vivo.* Thus, this indicates an immunomodulatory role of vMIP-II in directing inflammatory cell recruitment away from a T(H)1-type towards a T(H)2-type response and thereby facilitating evasion from cytotoxic reactions.

A selective leukocyte recruitment was demonstrated *in vivo* using chemokine receptor blockades as an *in vivo* model of acute inflammation. The role of RANTES/CCL5 and its receptors in the initiation and propagation of inflammation was investigated in rat models of acute allograft rejection. As a result, treatment with an antagonistic chemokine analogue (Met-RANTES) resulted in a dramatic reduction in acute inflammation in a moderately severe renal rejection model (Fisher RT1^{1v1} rat kidney into Lewis RT1¹ rat). Moreover, Met-RANTES profoundly suppressed vascular injury relative to untreated animals. Potential mechanisms of action of Met-RANTES were demonstrated in experiments using monocyte attachment assays on microvascular endothelium under physiological flow conditions. The results demonstrate that chemokine receptor antagonists can be used to dissect discrete stages in the initiation and propagation of inflammatory processes.

### Tumour Therapy

To determine the effect of the GPI-anchored chemokines in subjects having cancer, chemokines, with mucin stalk, are injected or administered subcutaneously, paratumourally, intravascularily to the site of tumour to effect leukocyte recruitment *in vivo.* Immune cells that were seen to be recruited include, for instance, leukocytes, T cells, monocytes, NK cells, and granulocytes such as eosinophils and neutrophils. The influx of specific leukocytes into the injection site can be evaluated using morphometry and immunohistochemistry. The systemic administration of chemokines generally leads to immune suppression wherein the regional expression of protein leads to selective recruitment. The recruitment of immune cells subsequently results in a cell-mediated cytotoxicity of tumour cells, and hence to the elimination of tumour cells in the affected tissue. The GPI-linked chemokines are therefore suitable agents for the treatment of a wide range of tissue cancers. As found by the inventors, by the addition of mucin the inhibitory effect on tumour growth and killing tumour cells can be further elevated.

For the treatment of cancer, the agents will be administered to the affected tissues in order generate defined surface concentrations of chemokine-mucin-GPI proteins within tissue environments. The fusion construct can be locally administered as anti-tumour adjuvant. Preferably, the fusion construct is administered at a concentration of 0,5 to 5 µg/ml, preferably 1 µg/ml. In one embodiment, the fusion construct is administered by spraying into the wound and/or injection into regions that are not available for surgery. This will foster the induction of leukocyte firm adhesion and spreading under physiological flow, transendothelial migration, and the induction of leukocyte effector function. As demonstrated in the examples, the chemokine-GPI constructs of the invention effect a reduction of tumour progression by recruiting immune cell such as leukocytes, T-cells, Natural Killer (NK) cells and neutrophils.

Tumours and cancer cells that may be treated with the GPI-anchored Chemokine include the following but not limiting cancers: breast cancer, renal cancer, prostate cancer, seminomas, melanomas, teratomas, neuroblastomas, gliomas, rectal cancer, endometrial cancer, kidney cancer, adrenal cancer, thyroid cancer, blood cancer, skin cancer, cancer of the brain, cervical cancer, intestinal cancer, liver cancer, colon cancer, stomach cancer, intestine cancer, gastrointestinal cancer, lymph node cancer, esophagus cancer, colorectal cancer, pancreas cancer, ear, nose and throat (ENT) cancer, cancer of the uterus, ovarian cancer and lung cancer.

Furthermore, the anti-tumour agents are also suitable for the treatment of residual cancer. Residual cancer often develops as a secondary tumour after surgery of the primary tumour and originates from tumour cells that could not be properly removed by the surgeon. Examples of residual cancer are breast cancer and glioblastoma (astrocytoma VI).

In conclusion, GPI-linked chemokines, with mucin domain, are suitable agents for an anti-tumour therapy. By anchoring the chemokine via the GPI-anchor into the surface membrane of tumour cells, malignant cells are eliminated by the action of immune cells.

### Tissue Regeneration and Suppression of Vascular Damage

Heterotopic heart transplant rejection models in the rat demonstrated that the RANTES/CCL5 receptor CCR1 appears to play a significant role in acute tissue damage. The CCR1 receptor antagonist BX 471 dramatically prolonged cardiac engraftment when used in conjunction with a low dose of cyclosporin. The mechanism of action of the CCR1 antagonist was studied in *in vitro* flow assays over microvascular endothelium. Accordingly, it was found that the antagonist blocks the firm adhesion of monocytes triggered by RANTES/CCL5 on inflamed endothelium.

The perfusion of a GPI-anchored chemokine based antagonist through the organ prior to transplantation allows the insertion of the GPI anchor into the microvascular endothelial cell membranes and thus presentation of the antagonist to the circulating leukocytes. The approach provides the protection to the vasculature of the allograft during the critical first few days after transplantation and thus significantly limits the acute vascular damage associated with poor prognosis for transplant engraftment.

As an example, Met-RANTES treatment was shown to dramatically reduce acute and chronic tissue damage in mouse models of heart transplantation as well as in acute and chronic rat renal allograft rejection models. The mechanisms of action of Met-RANTES and CCL5 receptors were demonstrated in experiments using monocyte and CD4+ T cell attachment assays on microvascular endothelium under physiological flow conditions where leukocyte firm adhesion was shown to be suppressed by the antagonist. This effect leads *in vivo* to a pronounced suppression of vascular injury in the Met-RANTES treated animals relative to untreated animals. Limiting this early damage to vessels was linked to a general suppression in the development of chronic allograft rejection. Thus, chemokine receptor antagonists can influence discrete stages in the initiation and propagation of inflammatory processes and represent important targets for therapeutic intervention.

A NMR analysis of the structure of CCL5 shows that the amino terminus is unconstrained and is presented out from the core protein. The integrity of the amino terminus of CCL5 is crucial for receptor binding and cellular activation. The extension of human CCL5 by a single methionine residue at the amino terminus is sufficient to produce a potent and selective antagonist. The carboxyl terminus is well suited for a GPI anchor allowing apical presentation of the antagonistic amino terminus from the endothelial surface. In addition, there is a clear difference in the signaling properties of aggregated and disaggregated CCL5 forms. The CCL5 protein forms higher order oligomers that have been shown to be essential for CCR1-mediated arrest. Single charged residues at either position 26 or 66 play key roles in this aggregation. CCL5 mutants deficient in oligomerization have been shown to block recruitment of monocytes and CD45RO+ CD4+ T cells triggered by RANTES immobilized on activated endothelium under flow conditions.

To generate a GPI anchored version of a CCL5-receptor antagonist two additional modifications were introduced into the CCL5 sequence. The first was the addition of an N terminal methionine interspersed between the signal sequence and mature protein. Second, a mutation of the glutamic acid residue at amino acid 66 to an alanine which has been shown to render the protein dimeric. These changes insured that the resultant protein is antagonistic. In addition, the resultant protein is easier to work with as a non-aggregating version of the protein, and thus is easier to purify, quantify and administer. The purified protein was found to be efficiently incorporated into the surface of endothelial cells and to block the transendothelial migration of a monocyte cell line. The fusion constructs of the present invention therefore represent a viable alternative to the systemic administration of recombinant antagonists for the suppression of acute vascular damage to allografts.

In summary, antagonistic GPI-linked chemokines are useful as tumour immune adjuvants, and can be applied to cells *in vitro* or tissue *in vivo* to be incorporated in the surface membranes of cells. In addition, beside their anti-tumour activity, the GPI-linked chemokines have been proved to be useful in tissue regeneration and suppression of acute vascular damage to allografts.

### BRIEF DESCRIPTION OF THE DRAWINGS

**Figure 1****. Chemokines and chemokine receptors in recruitment and activation of effector leukocytes.**
   The figure exemplifies how recruitment of leukocytes is achieved by chemokine response in vascular tissue. Effector leukocytes such as monocytes and T cells are recruited to the site of inflammation by the action of chemokines such as TNF-alpha and IL-1 beta. Efficient leukocyte recruitment to sites of inflammation requires the selectin family of adhesion molecules such as I-CAM, V-CAM and E-selectin. Once leukocytes are captured, they may transiently adhere to the venola endothelium and begin to roll. This process is called "leukocyte rolling". Rolling occurs at or below the velocity of freely rolling cells like erythrocytes in the same vessel and the same radial position. The selectin family of transmembrane adhesion receptors mediates this rolling process.
**Figure 2****. GPI anchor expression system.**
   An example of an expression vector containing the cDNA sequence encoding for a chemokine followed by a GPI signal sequence LFA3 is shown to be used for stable expression in CHO cells. The concept of GPI-anchoring proteins is illustrated. As control, His-tagged GPI-anchored proteins can be detected and purified. The GPI-anchored chemokine-mucin protein construct according to the present invention are incorporated in the surface membrane for adhesion.
**Figure 3****. Chemokine presentation on endothelial surface.**
   The figure illustrates the basic principle of binding of different GPI constructs according to the invention in the surface membrane of cells.
**Figure 4****. GPI anchor - mucin/chemokine.**
   A construct consisting of chemokine-mucin GPI is constructed and added to cells to be incorporated in their surface membrane. These constructs are successful for treatment of cancer since they facilitate the selective recruitment of leukocytes to the site of tumor. The mucin stocks incorporated in these constructs act to promote adhesion of the leukocyte cells triggered by the chemokine agent.
**Figure 5****. Example of an expression vector for a Chemokin-mucin-GPI fusion construct.**
   Chemokine genes and chemokine mucin domains were amplified from cDNA-chemokine templates using RT-PCR and oligonucleotides with engineered compatible cloning sites. Following DNA sequencing, the regions were cloned into the pEF vector and introduced into CHO cells by electroporation. Selection and amplification were performed as described (Mack et al. P.N.A.S. 92:7021,1995). Site mutations have been introduced into the chemokine genes using Stratagene - QuikChange® Site-Directed Mutagenesis Kit. The number of nucleotides and amino acids for each coding sequence are indicated.
**Figure 6****. Purification of a chemokine-GPI-construct (RANTES-GPI) using FPLC**
   Proteins were extracted using the indicated reagents and conditions, and the presence of proteins was confirmed by Silver stain. Desalted proteins extracts were loaded onto a Heparin Sepharose fast flow column, and RANTES-GPI was eluated with increasing concentrations of NaCl.

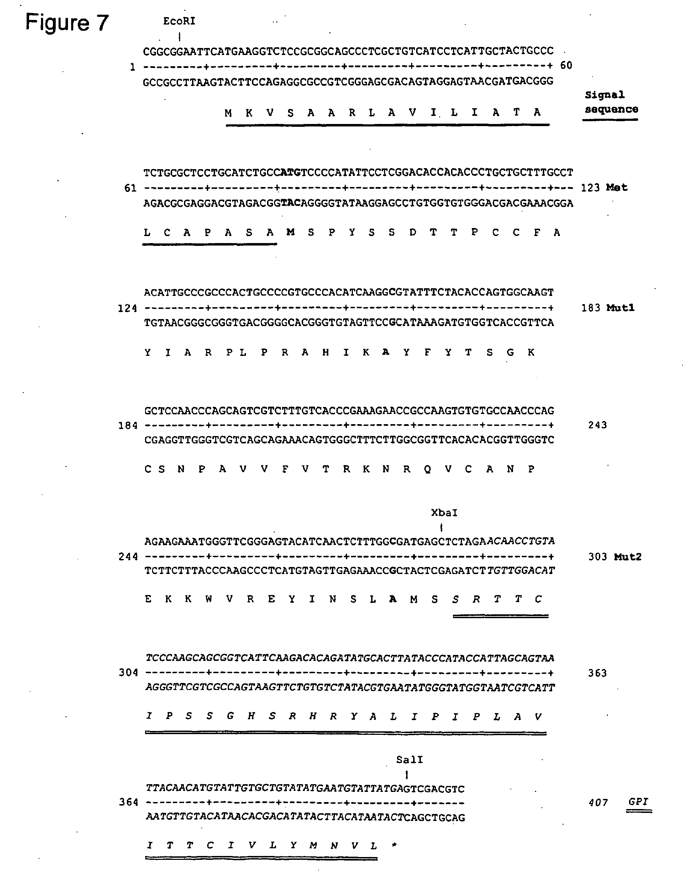

**Figure 7****. Generation of GPI anchored CCL5 variants**
   Scheme showing the sequence of CCL5 detailing the various modifications introduced to generate GPI-anchored versions of the protein. (Met-CCL5(tetramer)-GPI): single amino acid substitution at amino acid 26 (Mut 1) (glutamic acid for serine) renders the resultant RANTES protein to a tetramer. In a second construct Met-CCL5(dimer)-GPI: single amino acid substitution at amino acid 66 (Mut 2) (glutamic acid for serine) renders the resultant RANTES protein to a dimer. The GPI anchor signal sequence from LFA-3 was fused to the 5' end of CCL5 cDNA lacking a stop codon.

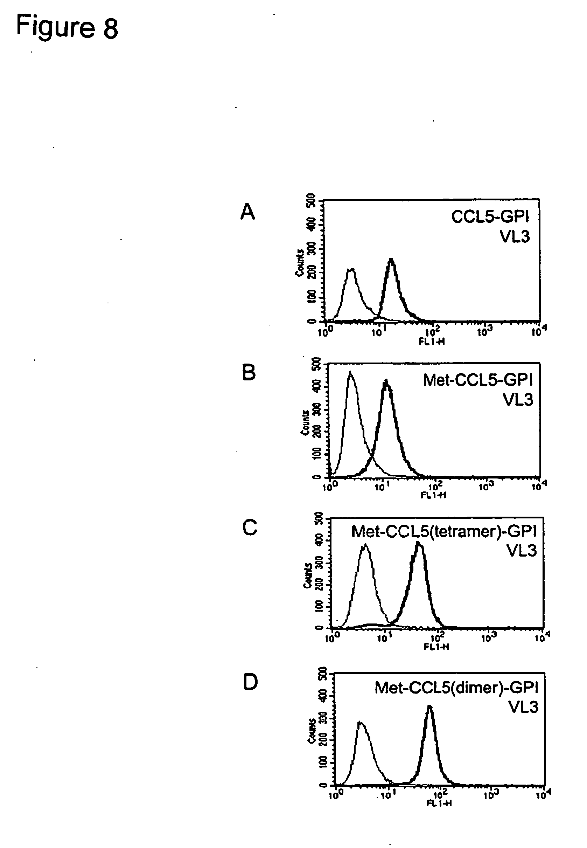

**Figure 8****. Expression of CCL5-GPI variants in CHO cells**
   The GPI-anchored modifications of CCL5 were over-expressed in CHO cells. FACS plot of stably transfected CHO cells using the anti-human CCL5 monoclonal antibody VL3 demonstrates cell surface expression of the proteins. (A) CCL5-GPI, (B) Met-CCL5-GPI (C) Met-CCL5(tetramer)-GPI and (D) Met-CCL5(dimer)-GPI.

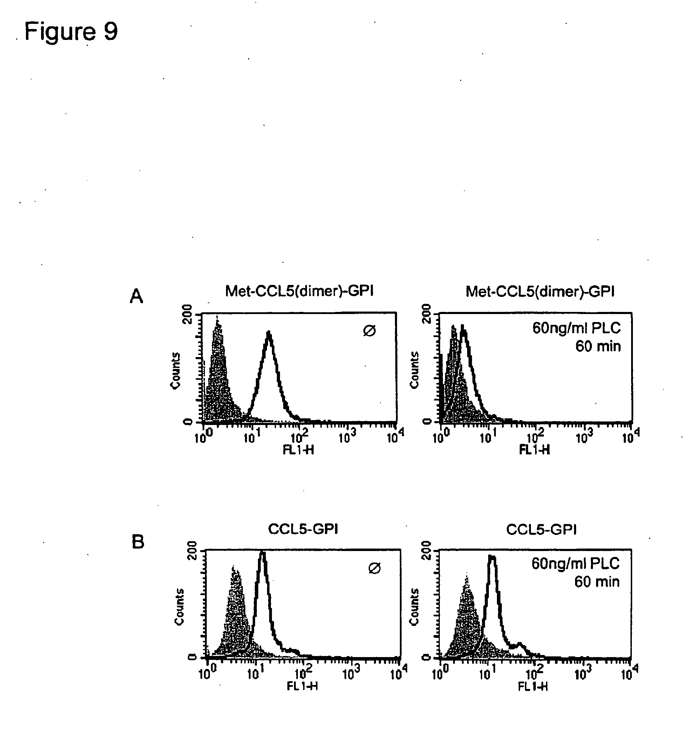

**Figure 9****. Cleavage of GPI anchor by PLC digestion**
   (A) Met-CCL5(dimer)-GPI and (B) CCL5-GPI expressing CHO cells were treated with 120 ng/ml phosphatidylinositol-specific phspholipase C for 60 min before FACS analysis. CCL5 and Met-CCL5 released into was measured in the supernatant using a human RANTES/CCL5 specific ELISA.

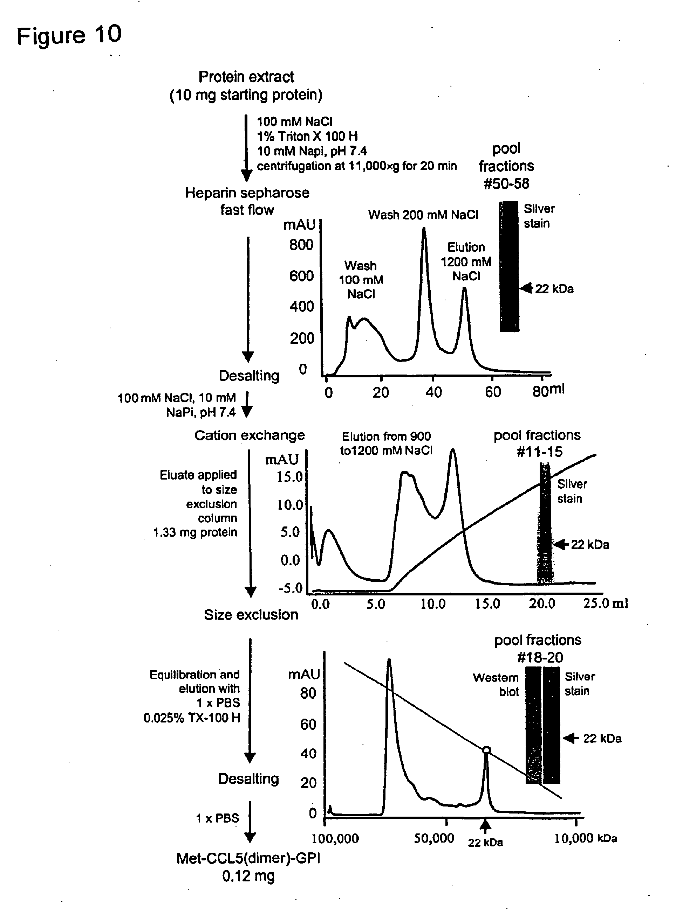

**Figure 10****. Purification of Met-CCL5(dimeric)-GPI anchored fusion protein**
   Outline of the procedure used to purify the Met-CCL5(dimer)-GPI protein from CHO over expressing cells using fast protein liquid chromatography (FPLC). Protein was detected by silver stain and Met-CCL5(dimer)-GPI protein by Western Blot using the anti-human CCL5 monoclonal antibody VL3 (Nelson, 2000; von Luettichau et al., 1996).

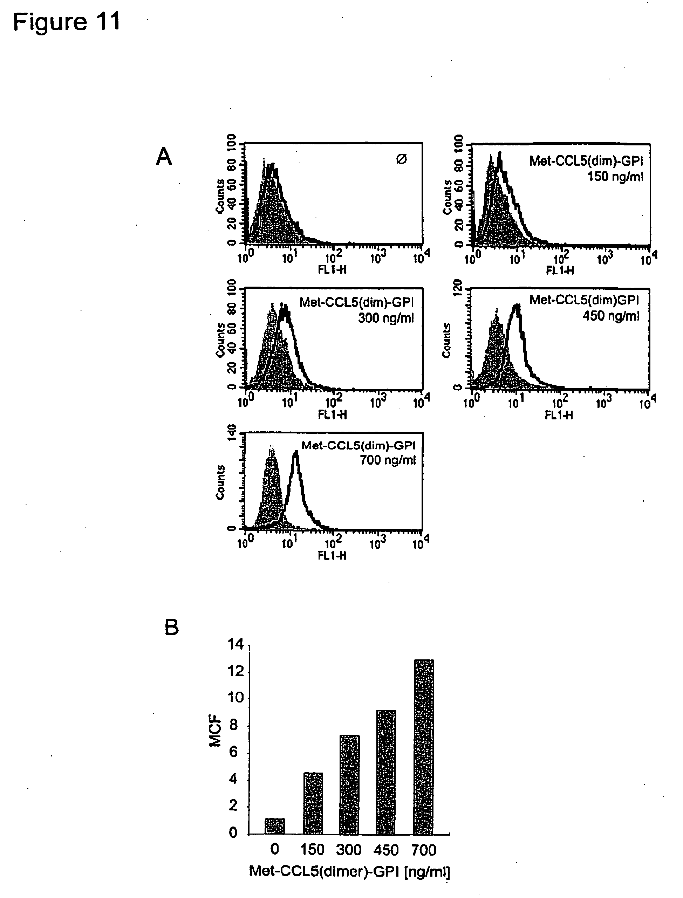

**Figure 11****. Incorporation of Met-CCL5(dimer)-GPI into endothelial cell membranes**
   To demonstrate reincorporation of the purified Met-CCL5(dimer)-GPI protein into endothelial cell membranes, the purified protein was added to HuMVEC and incubated at 37°C for 30 min. Met-CCL5(dimer) was detected on the cell surface by FACS analysis using VL3 (Nelson, 2000; von Luettichau et al., 1996). (A) FACS of endothelial cells after addition of increasing concentrations of Met-CCL5(dimer)-GPI as shown. (B) Plot of Mean Channel Florescence vs. concentration of Met-CCL5(dimer)-GPI used demonstrates a dose dependant incorporation of the GPI-anchored protein into the cell surface.

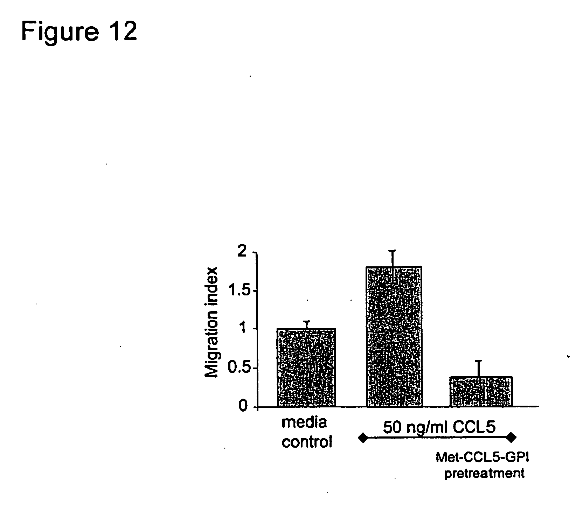

**Figure 12****. Suppression of transendothelial migration of monocyte cells by Met-CCL5(dimer)-GPI**
   Pretreatment of microvascular endothelial cells with 100 ng/ml Met-CCL5-GPI effectively suppressed transendothelial migration of monocyte cells induced by 50 ng/ml CCL5. Treatment also appeared to reduce the background migration of the THP-1 cells to media control. Representative result shown was taken from three independent experiments.

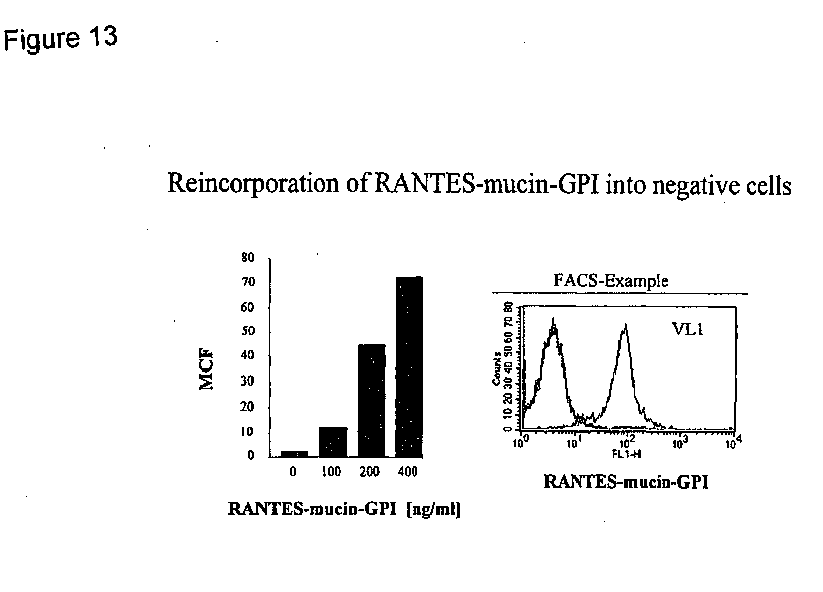

**Figure 13****. Reincorporation of RANTES-mucin-GPI into negative cells.**
   To demonstrate reincorporation of the purified RANTES-mucin-GPI protein into endothelial cell membranes, the purified protein construct was added to HuMVEC and incubated at 37°C for 30 minutes. RANTES-mucin-GPI was detected on the cell surface by FACS analysis using VL3 (Nelson, 2000; von Luettichau et al., 1996). (A) FACS of endothelial cells after addition of increasing concentrations of RANTES-mucin-GPI as shown. (B) Plot of Mean Channel Florescence vs. concentration of RANTES-mucin-GPI used demonstrates a dose dependant incorporation of the GPI-anchored protein into the cell surface.
**Figure 14****. Nucleic acid sequence of the IP-10-(fractalkine mucin domain)-GPI construct.**
   A chimeric construct of the invention comprising the chemokine IP-10, the mucin domain of fractalkine followed by GPI-anchor was constructed and sequenced. As a result, a nucleic acid sequence was obtained comprising the nucleic acid residues 1 to 1165.

### EXAMPLES

### Example 1 (reference example)

### Construction and purification of the CCL5-GPI fusion protein

Human CCL5 was cloned from cDNA (Schall et al., 1990) using hCCL5 specific primers and PCR. The CCL5 cDNA sequence (without a translation stop codon) was fused to a GPI signal sequence cloned from LFA-3 cDNA (Kirby et al., 1995).

Modifications were then introduced into the CCL5-GPI construct. A methionine residue was added to the amino terminal of the mature protein, just after the signal sequence. Met-CCL5-GPI was then combined with either the E66A or E26A mutation to generate non-aggregating, functional antagonists fused to a GPI anchor. The resulting constructs were then subcloned into the pEF-DHFR vector and stably introduced into CHO cells (Mack et al., 1995). Surface human CCL5 antigen expression was determined using FACS analysis and the hCCL5 specific antibody VL3 (Nelson, 2000; von Luettichau et al., 1996; Figure 8 A-D). VL3 was selected from a panel of previously characterized anti-CCL5 monoclonal antibodies (Nelson, 2000; von Luettichau et al., 1996). Unlike many of the other anti-CCL5 monoclonal antibodies tested, the modifications made to the CCL5 protein did not disrupt the epitope recognized by the VL3.

### Cell culture

Chinese hamster ovary cells deficient in DHFR (CHO/dhfr-) (ATCC, Rockville, MD, USA No. CRL.9096) were cultured in complete a-medium (GIBCO BRL, Life Technologies GmbH, Eggenstein, Germany No. 32561-029) supplemented with 10% heat-inactivated dialyzed FCS (GIBCO BRL, Life Technologies GmbH, Eggenstein, Germany No. 26400-044) and HT supplement (GIBCO BRL, Life Technologies GmbH, Eggenstein, Germany No. 41065-02). Human microvascular endothelial cells were obtained from Promocell (Heidelberg, Germany) and cultured in the media provided. The THP-1 monocyte cell line was obtained from the American Type Culture Collection and cultured in RPMI 1640 medium (GIBCO BRL, Life Technologies GmbH, Eggenstein, Germany) supplemented with 2 mM L-glutamine (Biochrom KG, Berlin) and 10% heat-inactivated FCS (Biochrom KG, Berlin, No. S0115). Fresh medium was given every third day and cultures were split when cells were confluent. Recombinant CCL5 protein (#278-RN) was obtained from R&D Systems (Minneapolis MN, USA).

### Construction and stable expression of GPI-anchored CCL5 modifications in CHO/dhfr-

The GPI-anchor attachment sequence of LFA-3 (Kirby et al., 1995) was amplified by PCR and cloned into the pEF-DHFR vector (Bacon et al., 1995; Djafarzadeh et al., 2004). The sense primer corresponded to nucleotides 616-633 of LFA-3 and contains an integrated XbaI restriction site to facilitate subcloning (underlined) (5'-TCTTTGGAGARGAGCTCTAGAACAACCTGTATCCCAAGC AG-3'). The antisense primer corresponded to nucleotides 860-877 of LFA-3 and included a SalI site (underlined) (5'CCCGCGGCCGCTATTGGCCGACGTCGACTCATAATACATTCATATACAGCACA ATACATGTTG-3'). The DNA fragment encoding hCCL5 but excluding the stop codon was amplified by PCR from cDNA (Schall et al., 1988). The sense primer included the initiation codon for human CCL5 with a modification to include an EcoRI restriction site (underlined) (Forward Primer: 5'-CGGCGGAATTCATGAAGGTCTCCGCGG-3'). The antisense primer introduced an XbaI site (underlined) (Reverse Primer: 5'-TGGGATACAGGTTGTTCTAGAGCTCATCTCCAAAGAGTTGATG-3'). The GPI anchor signal sequence and fusion contributed nine additional amino acids to the carboxyl end of CCL5 (SSRTTCIPS). The amplified gene sequences were then subcloned sequentially into the pEF-DHFR vector and stably introduced into CHO(dhfr-) using electroporation as described (Mack et al., 1995).

Modifications of the CCL5 sequence included the introduction of a N-terminal methionine between the signal sequence and mature protein, and the exchange of the glutamic acid at amino acid 66 for alanine (E66A), the dimer mutation, and the exchange of glutamic acid 26 for alanine (E26A) rendering the resultant protein a tetramer (Figure 7; Czaplewski et al., 1999). The mutations were generated by site directed mutagenesis using the QuickChangeTM Site-Directed Mutagenisis Kit (StratageneR Catalog #200518) used according to the manufacture's directions. The primers used were: Met-CCL5 forward primer: 5'-CCTGCATCTGCCATGTCCCCATATTCCTCG-3', reverse primer: 5'-GGACGTAGACGGTACAGGGGTATAAGGAGC-3'; E66A forward primer: 5'-CGTGCCCACATCAAGGCGTATTTCTACACCAG-3', reverse primer: 5'-CTGGTGTAGAAATACGCCTTGATGTGGGCACG-3'and; E26A forward primer: 5'-GTACATCAACTCTTTGGCGATGAG CTCTAGAACAACC-3' reverse primer: 5'-GGTTGTTCTAGAGCTCATCGCCAAAGAGTTGATGTAC-3'.All mutants were generated in pUC 18 (Yanisch-Perron et al., 1985) and DNA sequenced prior to subcloning.

### Fluorescence activated cell sorting (FACS) analysis

Expressed human CCL5 and its variants were identified on the CHO cell surface by FACS analysis. Cells were detached with 1.5 mM EDTA (Biochrom A, Berlin, Germany, No. L2113) in 1 x PBS and incubated for 60 min on ice with the CCL5 antibody VL3 or VL4 (Nelson, 2000; von Luettichau et al., 1996) (5 µg/ml) or IgG2bk isotype control (Sigma # M-8894) (5 µg/ml). The cells were washed three times with 1 x PBS and incubated with a FTIC-conjugated donkey anti-mouse (DAKO A/S, Denmark No. F0313) IgG for 45 min on ice. The cells were washed three times with 1 x PBS and analyzed on a flow cytometer (FACS Calibur, Becton, Dickinson and Company, USA) using CellQuest analysis software.

### Purification of Met-CCL5(dimer)-GPI protein

The cells were washed three times with cold 1 x PBS and detached with 1.5 mM EDTA. The cells were rotated 1 h at 4°C with hypertonic lysis buffer (5 sodium phosphate, 2 mM MgCl, 0.1 mM EDTA, protease inhibitor cocktail tablets (Roche, Basel, Switzerland No. 1697498). Cell membranes were isolated with extraction buffer [100mM NaCl 1% Triton X-100 Hydrogenated (Triton X-100 H) (CALBIOCHEM, Merck Darmstadt, Germany No 648464) 10 mM sodium phosphate, 5 mM EDTA, protease inhibitor cocktail tablets, pH 7.4] and were rotated for 1 h at 4 °C followed by centrifugation at 11,000g for 20 min at 4°C. The supernatant was used for further purification using FPLC.

Heparin affinity chromatography (Heparin Sepharose Fast Flow, Amersham Biosciences, Sweden) was used for the initial column purification step. The column was equilibrated with 100mM NaCl, 10 mM sodium phosphate, 0.1% Triton X-100 H pH 7.4 and the Met-CCL5(dimer)-GPI protein was eluted from Heparin column with 1200 mM NaCl, 0.1% Triton X-100 H, 10 mM sodium phosphate, pH 7.4. The eluted fractions containing Met-CCL5(dimer)-GPI were pooled and the buffer changed using a desalting column (HiTrap Desalting, Amersham Biosciences, Uppsala, Sweden No. 17-1408-01).

A cation exchanger column (SP Sepharose HP, Amersham Biosciences, Uppsala, Sweden No. 17-1087-01) was used as the next chromatography step. The column was equilibrated with 100mM NaCl, 10 mM sodium phosphate, 0.1% Triton X-100 H pH 7.4 and the Met-CCL5(dimer)-GPI protein was eluted from the cationic exchange column using a salt gradient from 900 to 1200 mM NaCl, 0.1% Triton X-100 H, 10 mM sodium phosphate, pH 7.4. The protein pool was concentrated 20 fold using a Centricon concentrator Amicon Ultra with pore size of 10,000 MWCO (Millopore, Eschborn, Germany No. UFC 901024). The concentrated protein was then applied to a gel filtration TSK column G3000SWXL (TOSOH Corporation, Tokyo, Japan No. CHO-1252) column using 1 x PBS and 0.025 % Triton X-100 H. The final step used a desalting column equilibrated with 1 x PBS to decrease the concentration of Triton X-100 H. Met-CCL5(dimer) protein was detected in column fractions by Western blot analysis using VL3 monoclonal antibody (von Luettichau et al., 1996).

### CCL5 ELISA

A human CCL5 specific ELISA kit was used to detect Met-CCL5(dimer) in solution using the RANTES/CCL5 protocol applied according to the manufacture's directions (R&D Systems). The capture anti-human CCL5 mAB (840216), biotinylated goat anti-human CCL5 detection antisera (840217), Strepavidin-HRP (890803) and rhCCL5 protein (840218) were purchased from R&D Systems GmbH (Wiesbaden, Germany).

### Example 2 (reference example)

### PLC digestion confirmed GPI anchorage of CCL5

GPI anchorage of the Met-CCL5(dimer)-GPI protein was confirmed following PI-PCL (phosphatidylinositol-specific phospholipase) digestion. Stably expressing CHO cells were treated with 120 ng/ml phosphatidylinositol-specific phospholipase C (PLC) (SIGMA-ALDRICH, Taufkirchen, Germany No. 661-9) in serum-free medium for 60 min at 37°C and 5 % CO2 and subjected to FACS analysis. CCL5 FACS using VL3 antibody demonstrated loss of surface signal that correlated with digestion (Figure 9 A). Interestingly, the native CCL5-GPI linked protein proved resistant to PLC digestion (Figure 9 B) suggesting that the oligomeric version of the protein may not allow adequate access to the PLC enzyme and that partial digestion of the GPI anchors linking the protein to the surface may not allow efficient release of aggregated protein from the membrane.

### Example 3 (reference example)

### Purification of the CCL5-GPI fusion protein

CCL5-GPI-fusion protein was purified from the transfected cells by Triton X-100 detergent extraction followed by column purification using heparin sepharose, cationic exchange and size exclusion chromatography. The general purification scheme is outlined in Figure 10. The presence of the Met-CCL5(dimer)-GPI protein was followed by Western blot analysis using the VL3 antibody (von Luettichau et al., 1996) and silver stain (Figure 10).

### Example 4 (reference example)

### Exogenously added CCL5-GPI-anchored protein is incorporated into cell membranes in a dose dependant manner

To demonstrate reincorporation of the GPI-CCL5 protein into cell membranes 150, 300, 450 and 700 ng/ml of purified Met-CCL5(dimer)-GPI protein was added to microvascular endothelial cells in cell culture, incubated for 30 min at 37°C. The cells were then washed three times and subjected to FACS analysis (Figure 11 A). The addition of increasing concentrations of Met-CCL5(dimer)-GPI shows a dose dependent incorporation of the GPI-anchored protein into the endothelial cell surface as evidenced by FACS (Figure 11 B).

The efficiency of reincorporation of the Met-CCL5(dimer)-GPI protein was determined using two approaches. In the first, a specific concentration of GPI anchored agent (700 ng/ml) was incubated with endothelial cells as described in Figure 11 A. The cells were washed, and the amount of protein left in the wash supernatant was determined using a human RANTES specific ELISA assay. In the second approach, following reincorporation using 700 ng/ml of protein into endothelial cells, the cells were washed, and the GPI anchor was cleaved by treatment with 120 ng/ml PLC. The cells were washed again and the freed protein present in the supernatant was measured using human RANTES ELISA. Using the first method the efficiency of incorporation was determined to be 52% of starting material. Using the PLC digestion/ELISA approach, 24% of starting protein was recovered after digestion. Based on these two methods between 25 and 50 % of 700 ng/ml starting material is incorporated into cell membranes. This is similar to the efficiency of incorporation calculated for other GPI-anchored proteins (Djafarzadeh et al., 2004).

It was previously demonstrated that MetRANTES is a functional antagonist of the chemokine receptors CCR1, CCR3 and CCR5 (Proudfoot et al., 1999; Grone et al., 1999; Proudfoot et al., 1996). In addition, the aggregation mutant (dimer E66A) has also been shown to be an antagonist for CCR1 (Baltus et al., 2003). Functionality of the Met-CL5(dimer)-GPI protein was demonstrated using transendothelial migration assays utilizing a modified Boyden Chamber assay and primary human microvascular endothelial cells (HMVC) (Weber et al., 2001). Briefly, Primary dermal microvascular endothelial cells, HMVC, were grown to confluency on Costar (#3472) 24 well Transwell inserts, pore size of 3 um and diameter of 6.5 Transendothelial migration of the monocyte cell line was optimal at 50 ng/ml rhCCL5 (data not shown) (Weber et al., 2001). THP-1 cells were labelled with 10 µg/ml Calcein (Molecular Probes, Eugene, Oregon, USA) and suspended at 2x105 in 100 µl of assay media (RPMI 1640, 0.1% BSA and 10mM HEPES). The cells were placed in triplicate in the upper wells of the chamber and media control or recombinant human CCL5 in the lower chamber (0, 50 and 100 ng/ml) (600 µl assay media). Plates were incubated for 2 hours at 37°C and increase of Fluorescence was measured at emission of 535 nm, excitation 485 nm using a TECAN GENios Plus ELISA reader (TECAN, Grödig, Austria) and XFluor 4 software (TECAN, Grödig, Austria). The migration index was evaluated against control. The results represent two independent experiments performed in triplicates as described (Djafarzadeh et al., 2004).

### Example 5 (reference example)

### Suppression of acute vascular damage by blocking CCL5 receptors

To test the ability of the Met-CCL5(dimer)-GPI agent to block CCL5 receptors, the confluent HMVC/Transwells were incubated for 30 min at room temperature with 100 ng/ml Met-CCL5(dimer)-GPI which was then washed two times with media lacking FCS. Endothelial cells so treated, effectively blocked the transmigration of monocyte cell line to CCL5 signal and in addition also showed a reduced background transmigration of the cells to media control (Figure 12).

### Example 6 (reference example)

### Application of the chemokine-GPI agents in vivo

The fusion constructs as indicated in Table 1 were tested as agents both in tumour models *in vitro* and *in vivo.* GPI-linked chemokine were administered at a concentration of 1 µg/ml at the tumour tissue in a subject to generate defined surface concentrations of chemokine-mucin-GPI proteins within tissue environments. The application of GPI-chemokine caused the induction of leukocyte firm adhesion and spreading under physiological flow, transendothelial migration, and the induction of leukocyte effector function. In a next step, the effects on tumour progression and immune response have been determined. As a result, the addition of a chemokine-GPI agent resulted in a significant regression of tumour growth.

### Example 7 (reference example)

### Leukocyte recruitment in vivo following injection of Chemokine-GPI.

Chemokines without mucin stalk as indicated in Table 1 were injected or administered subcutaneously, paratumourally, or intravascularily to effect the recruitment of leukocytes (T cells, monocytes, NK cells, eosinophils and neutrophils). The influx of specific leukocytes into the injection site was evaluated using morphometry and immunohistochemistry. A systemic administration of chemokines generally resulted in an immune suppression. Moreover, the regional expression of the fusion construct protein resulted in a selective recruitment of leukocytes.

### Example 8

### Leukocyte recruitment in vivo following injection of Chemokine-mucin-GPI.

A series of chemokine-mucin-GPI fusion proteins have been constructed and tested for the capability in leukocyte recruitment both *in vitro* and *in vivo.* The proteins prepared include a series of human RANTES/CCL5 based agents, human and murine IP-10/CXCL10 based proteins, and fusion constructs utilizing human IL-8/CXCL8.

GPI-linked chemokines with mucin stalk as indicated in Table 1 were injected or administered subcutaneously, paratumourally, or intravascularily similar to Example 7. In the experiment as shown in Figure 13, injection of RANTES-mucin-GPI and testing of enhanced selective leukocytes is performed using both endogenous leukocytes as well as adaptively transferred lymphocytes (e.g. anti-tumour T cells). The presence of a mucin domain in the RANTES-GPI construct resulted in an efficient incorporation of the chemokine agent into the surface membrane of a cell (see Figure 13). This resulted in an increased recruitment and adhesion of leukocyte cells (T cells, monocytes, NK cells, eosinophils and neutrophils) to the tumour site. Subsequently, the effect of leukocyte recruitment on tumour growth has been determined. It was found that tumour growth is significantly decreased by RANTES-mucinchemokine and the other tested chemokine-mucin-GPI constructs. In other experiments, the co-injection of stem cells (expressing complementary receptors) in the context of GPI-chemokine administration was tested to enhance the specific accumulation of stem cells at a select tissue site.

These results suggest that the efficiency of chemokine-GPI anchoring can be further enhanced by including one or more mucin domains in the fusion constructs of the invention. In addition, the results confirm that the chemokine-mucin-GPI construct is an efficient agent for the treatment of cancer by recruitment of leukocytes.

### REFERENCES

Adam, J. K., Odhav, B., and Bhoola, K. D. (2003) Immune responses in cancer. Pharmacol Ther 99, 113-132.
Bacon, K., Baggiolini, M., Broxmeyer, H., Horuk, R., Lindley, I., Mantovani, A., Maysushima, K., Murphy, P., Nomiyama, H., Oppenheim, J., Rot, A., Schall, T., Tsang, M., Thorpe, R., Van Damme, J., Wadhwa, M., Yoshie, O., Zlotnik, A. and Zoon, K. (2002). J Interferon Cytokine Res 22, 1067-1068.
Bacon, K., Baggiolini, M., Broxmeyer, H., Horuk, R., Lindley, I., Mantovani, A., Maysushima, K., Murphy, P., Nomiyama, H., Oppenheim, J., Rot, A., Schall, T., Tsang, M., Thorpe, R., Van Damme, J., Wadhwa, M., Yoshie, O., Zlotnik, A., and Zoon, K. (2002) Chemokine/chemokine receptor nomenclature. J Interferon Cytokine Res 22, 1067-1068.
Bacon, K. B., Premack, B. A., Gardner, P. and Schall, T. J. (1995). Science 269, 1727-1730.
Baltus, T., Weber, K. S., Johnson, Z., Proudfoot, A. E. and Weber, C. (2003). Blood 102, 1985-1988.
Bedke, J., Stojanovic, T., Grone, H. J., Heuser, M., Scheele, L., Proudfoot, A. E., Becker, H., Markus, P. M. and Hecker, M. (2002). Transplant Proc 34, 1049.
Bernardini, G., Ribatti, D., Spinetti, G., Morbidelli, L., Ziche, M., Santoni, A., Capogrossi, M. C., and Napolitano, M. (2003) Analysis of the role of chemokines in angiogenesis. J Immunol Methods 273, 83-101.
Borish, L. C., and Steinke, J. W. (2003) 2. Cytokines and chemokines. J Allergy Clin Immunol 111, S460-475.
Chapman, G. A., Moores, K. E., Gohil, J., Berkhout, T. A., Patel, L., Green, P., Macphee, C. H., and Stewart, B. R. (2000) The role of fractalkine in the recruitment of monocytes to the endothelium. Eur J Pharmacol 392, 189-195.
Coussens, L. M., and Werb, Z. (2002) Inflammation and cancer. Nature 420, 860-867.
Czaplewski, L. G., McKeating, J., Craven, C. J., Higgins, L. D., Appay, V., Brown, A., Dudgeon, T., Howard, L. A., Meyers, T., Owen, J., Palan, S. R., Tan, P., Wilson, G., Woods, N. R., Heyworth, C. M., Lord, B. I., Brotherton, D., Christison, R., Craig, S., Cribbes, S., Edwards, R. M., Evans, S. J., Gilbert, R., Morgan, P., Hunter, M. G. and et al. (1999). J Biol Chem 274, 16077-16084.
D'Ambrosio, D., Panina-Bordignon, P., and Sinigaglia, F. (2003) Chemokine receptors in inflammation: an overview. J Immunol Methods 273, 3-13.
Djafarzadeh, R., Mojaat, A., Vicente, A. B., von Luttichau, I. and Nelson, P. J. (2004). Biol Chem 385, 655-663.
Dong, V. M., McDermott, D. H., and Abdi, R. (2003) Chemokines and diseases. Eur J Dermatol 13, 224-230.
Dranoff, G. (2003) Coordinated tumour immunity. J Clin Invest 111, 1116-1118.
Grone, H. J., Weber, C., Weber, K. S., Grone, E. F., Rabelink, T., Klier, C. M., Wells, T. N., Proudfood, A. E., Schlondorff, D. and Nelson, P. J. (1999). Faseb J 13, 1371-1383.
Haskell, C. A., Cleary, M. D., and Charo, I. F. (2000) Unique role of the chemokine domain of fractalkine in cell capture. Kinetics of receptor dissociation correlate with cell adhesion. J Biol Chem 275, 34183-34189.
Haskell, C. A., Ribeiro, S. and Horuk, R. (2002). Curr Opin Investig Drugs 3, 399-405.
Hirose, K., Hakozaki, M., Nyunoya, Y., Kobayashi, Y., Matsushita, K., Takenouchi, T., Mikata, A., Mukaida, N., and Matsushima, K. (1995) Chemokine gene transfection into tumour cells reduced tumourigenicity in nude mice in association with neutrophilic infiltration. Br J Cancer 72, 708-714.
Kirby, A. C., Hill, V., Olsen, I. and Porter, S. R. (1995). Biochem Biophys Res Commun 214, 200-205.
Kooyman, D. L., Byrne, G. W. and Logan, J. S. (1998). Exp Nephrol 6, 148-51.
Kuna, P., Reddigari, S. R., Schall, T. J., Rucinski, D., Viksman, M. Y. and Kaplan, A. P. (1992). J Immunol 149, 636-642.
Kuna, P., Reddigari, S. R., Schall, T. J., Rucinski, D., Sadick, M. and Kaplan, A. P. (1993). J Immunol 150, 1932-1943.
Lucas, A. D., Chadwick, N., Warren, B. F., Jewell, D. P., Gordon, S., Powrie, F., and Greaves, D. R. (2001) The transmembrane form of the CX3CL1 chemokine fractalkine is expressed predominantly by epithelial cells in vivo. Am JPathol 158, 855-866.
Luster, A. D., and Leder, P. (1993) IP-10, a -C-X-C- chemokine, elicits a potent thymus-dependent antitumour response in vivo. J Exp Med 178, 1057-1065.
Luznik, L., Slansky, J. E., Jalla, S., Borrello, I., Levitsky, H. I., Pardoll, D. M., and Fuchs, E. J. (2003) Successful therapy of metastatic cancer using tumour vaccines in mixed allogeneic bone marrow chimeras. Blood 101, 1645-1652.
Mack, M., Riethmuller, G. and Kufer, P. (1995). Proc Natl Acad Sci U S A 92, 7021-7025.
Mackensen, A., Lindemann, A., and Mertelsmann, R. (1997) Immunostimulatory cytokines in somatic cells and gene therapy of cancer. Cytokine Growth Factor Rev 8, 119-128.
Mackensen, A., Veelken, H., Lahn, M., Wittnebel, S., Becker, D., Kohler, G., Kulmburg, P., Brennscheidt, U., Rosenthal, F., Franke, B., Mertelsmann, R., and Lindemann, A. (1997) Induction of tumour-specific cytotoxic T lymphocytes by immunization with autologous tumour cells and interleukin-2 gene transfected fibroblasts. J Mol Med 75, 290-296.
Maghazachi, A. A., al-Aoukaty, A. and Schall, T. J. (1994). J Immunol 153, 4969-4977.
Medof, M. E., Nagarajan, S. and Tykocinski, M. L. (1996). Faseb J 10, 574-586.
Murphy, P. M. (2001) Chemokines and the molecular basis of cancer metastasis. N Engl J Med 345, 833-835.
Murphy, P. M. (2002). International Union of Pharmacology. XXX. Update on chemokine receptor nomenclature. Pharmacol Rev 54, 227-229.
Murphy, P. M., Baggiolini, M., Charo, I. F., Hebert, C. A., Horuk, R., Matsushima, K., Miller, L. H., Oppenheim, J. J. and Power, C. A. (2000). International union of pharmacology. XXII. Nomenclature for chemokine receptors. Pharmacol Rev 52, 145-176.
Mule, J. J., Custer, M., Averbook, B., Yang, J. C., Weber, J. S., Goeddel, D. V., Rosenberg, S. A., and Schall, T. J. (1996) RANTES secretion by gene-modified tumour cells results in loss of tumourigenicity in vivo: role of immune cell subpopulations. Hum Gene Ther 7, 1545-1553.
Nakayama, T., Hieshima, K., Izawa, D., Tatsumi, Y., Kanamaru, A., and Yoshie, O. (2003) Cutting edge: profile of chemokine receptor expression on human plasma cells accounts for their efficient recruitment to target tissues. J Immunol 170, 1136-1140.
Nelson, P. J. (2000). Methods Mol Biol 138, 223-229.
Nelson, P. J. and Krensky, A. M. (1998). Curr Opin Immunol 10, 265-270.
Nelson, P. J. and Krensky, A. M. (2001). Chemokines, chemokine receptors, and allograft rejection. Immunity 14, 377-386.
Nelson, P. J., Pattison, J. M. and Krensky, A. M. (1997). Methods Enzymol 287, 148-162.
Ono, S. J., Nakamura, T., Miyazaki, D., Ohbayashi, M., Dawson, M., and Toda, M. (2003) Chemokines: Roles in leukocyte development, trafficking, and effector function. J Allergy Clin Immunol 111, 1185-1199.
Premkumar, D. R., Fukuoka, Y., Sevlever, D., Brunschwig, E., Rosenberry, T. L., Tykocinski, M. L. and Medof, M. E. (2001). J Cell Biochem 82, 234-245.
Proudfoot, A. E., Buser, R., Borlat, F., Alouani, S., Soler, D., Offord, R. E., Schroder, J. M., Power, C. A. and Wells, T. N. (1999). J Biol Chem 274, 32478-32485.
Proudfoot, A. E., Power, C. A., Hoogewerf, A. J., Montjovent, M. O., Borlat, F., Offord, R. E. and Wells, T. N. (1996). J Biol Chem 271, 2599-2603.
Schall, T. J., Bacon, K., Toy, K. J. and Goeddel, D. V. (1990). Nature 347, 669-71.
Schall, T. J., Jongstra, J., Dyer, B. J., Jorgensen, J., Clayberger, C., Davis, M. M. and Krensky, A. M. (1988). J Immunol 141, 1018-1025.
Sgadari, C., Angiolillo, A. L., and Tosato, G. (1996) Inhibition of angiogenesis by interleukin-12 is mediated by the interferon-inducible protein 10. Blood 87, 3877-3882.
Sgadari, C., Angiolillo, A. L., Cherney, B. W., Pike, S. E., Farber, J. M., Koniaris, L. G., Vanguri, P., Burd, P. R., Sheikh, N., Gupta, G., Teruya-Feldstein, J., and Tosato, G. (1996) Interferon-inducible protein-10 identified as a mediator of tumour necrosis in vivo. Proc Natl Acad Sci U S A 93, 13791-13796.
Skelton, N. J., Aspiras, F., Ogez, J. and Schall, T. J. (1995). Biochemistry 34, 5329-42.
Song, E., Zou, H., Yao, Y., Proudfoot, A., Antus, B., Liu, S., Jens, L. and Heemann, U. (2002). Kidney Int 61, 676-685.
Stojanovic, T., Bedke, J., Grone, H. J., Proudfoot, A. E., Becker, H., Markus, P. and Hecker, M. (2002). J Vasc Res 39, 51-58.
Tannenbaum, C. S., Tubbs, R., Armstrong, D., Finke, J. H., Bukowski, R. M., and Hamilton, T. A. (1998) The CXC chemokines IP-10 and Mig are necessary for IL-12-mediated regression of the mouse RENCA tumour. J Immunol 161, 927-932.
Umehara, H., Goda, S., Imai, T., Nagano, Y., Minami, Y., Tanaka, Y., Okazaki, T., Bloom, E. T., and Domae, N. (2001) Fractalkine, a CX3C-chemokine, functions predominantly as an adhesion molecule in monocytic cell line THP-1. Immunol Cell Biol 79, 298-302. von Hundelshausen, P., Weber, K. S., Huo, Y., Proudfoot, A. E., Nelson, P. J., Ley, K. and Weber, C. (2001). Circulation 103, 1772-1777.
von Luettichau, I., Nelson, P. J., Pattison, J. M., van de Rijn, M., Huie, P., Warnke, R., Wiedermann, C. J., Stahl, R. A., Sibley, R. K. and Krensky, A. M. (1996). Cytokine 8, 89-98.
Weber, C., Weber, K. S., Klier, C., Gu, S., Wank, R., Horuk, R. and Nelson, P. J. (2001). Blood 97, 1144-1146.
Wells, T. N., Proudfoot, A. E. and Power, C. A. (1999). Immunol Lett 65, 35-40.
Wiedermann, C. J., Kowald, E., Reinisch, N., Kaehler, C. M., von Luettichau, I., Pattison, J. M., Huie, P., Sibley, R. K., Nelson, P. J. and Krensky, A. M. (1993). Curr Biol 3, 735-739.
Wong, M. M., and Fish, E. N. (2003) Chemokines: attractive mediators of the immune response. Semin Immunol 15, 5-14.
Yanisch-Perron, C., Vieira, J. and Messing, J. (1985). Gene 33, 103-119.
Yun, J. J., Whiting, D., Fischbein, M. P., Banerji, A., Irie, Y., Stein, D., Fishbein, M. C., Proudfoot, A. E., Laks, H., Berliner, J. A. and Ardehali, A. (2004). Circulation 109, 932-7.
Zarour, H. M., and Kirkwood, J. M. (2003) Melanoma vaccines: early progress and future promises. Semin Cutan Med Surg 22, 68-75.

## Claims

1. A chemokine fusion construct comprising an amino acid sequence of a chemokine or a biologically active fragment thereof, wherein said chemokine or biologically active fragment thereof is linked to a mucin domain followed by a glycosylphosphatidylinositol (GPI)-anchor.

2. The fusion construct of claim 1, wherein said mucin domain is a membrane-bound mucin domain.

3. The fusion construct of claim 2, wherein said mucin domain comprises an amino acid sequence encoded by a gene selected from the group consisting of MUC1, MUC3A, MUC3B, MUC4, MUC11, MUC12, MUC16, and MUC17, or a portion thereof.

4. The fusion construct of claim 1, wherein said mucin domain comprises a mucin-stalk of the surface-associated chemokine CXCL16 or fractalkine (CX3CL1).

5. The fusion construct of any one of claims 1-4, wherein said chemokine is a member of the C, CC, CXC or CX₃C subfamily of chemokines.

6. The fusion construct of claim 5, wherein said chemokine C subfamily is selected from the group consisting of XCL1/Lymphotactin/SCM-1a, and XCL2/SCM-1β.

7. The fusion construct of claim 5, wherein said chemokine CC subfamily is selected from the group consisting of CCL1/I-309, CCL2/MCP-1, CCL3/MIP-1α, CCL3L1/LD78β,CCL4/MIP-1β,CCL5/RANTES, CCL6, CCL7/MCP3, CCL8/MCP-2, CCL9/CCL10, CCL11/Eotaxin, CCL12, CCL13/MCP-4, CCL14/HCC-1, CCL15/HCC-2/Lkn-1/MIP-1δ, CCL16/HCC-4/LEC/LCC-1, CCL17/TARC, CCL18/DC-CK1. CCL19/MIP-3β/ELC, CCL20/MIP-3α/LARC, CCL21/6Ckine/SLC, CCL22/MDC, CCL23/MPIF-1/CKb8, CCL24/Eotaxin-2, CCL25/TECK, CCL26/Eotaxin-3, CCL27/CTACK, CCL28/MEC.

8. The fusion construct of claim 5, wherein said chemokine CXC subfamily is selected from the group consisting of CXCL1/GROα, CXCL2/GROβ, CXCL3/GROγ, CXCL4/PF4, CXCL5/ENA-78, CXCL6/GCP-2, CXCL7/NAP-2, CXCL8/IL-8, CXCL9/Mig, CXCL10/IP-10, CXCL11/I-TAC, CXCL12/SDF-1α/β, CXCL13BCA-1, CXCL14/BRAK, CXCL15, CXCL16.

9. The fusion construct of any one of claims 1-5, wherein said chemokine is selected from the group consisting of fractalkine/CX₃CL1, CXCL16, IL-8/CXCL8, IL-10, IP-10/CXCL10, RANTES/CCL5.

10. The fusion construct of any one of claims 1-9, wherein said fusion construct contains one or more GPI signal sequences to direct GPI-anchoring.

11. The fusion construct of any one of claims 1-10, wherein said GPI-anchor is derived from the lymphocyte function-associated antigen (LFA-3), or a portion thereof.

12. The fusion construct of any one of claims 1-11, wherein said chemokine-mucin-GPI construct incorporates into the cell membranes of target cells.

13. A nucleic acid molecule comprising a nucleic acid sequence that encodes for the fusion construct according to any one of claims 1-12.

14. An expression plasmid comprising the nucleic acid molecule according to claim 13 and additional expression elements.

15. A host cell comprising the expression plasmid according to claim 14.

16. A vector comprising the nucleic acid molecule according to claim 13.

17. A pharmaceutical composition comprising the fusion construct according to any one of claims 1-12 or the nucleic acid molecule according to claim 13, and a pharmaceutically acceptable carrier.

18. Use of a polypeptide of a fusion construct according to any one of claims 1-12 for the preparation of a medicament for the treatment of cancer.

19. The use of claim 18, wherein said cancer is selected from the group consisting of breast cancer, renal cancer, prostate cancer, seminomas, melanomas, teratomas, neuroblastomas, gliomas, rectal cancer, endometrial cancer, kidney cancer, adrenal cancer, thyroid cancer, blood cancer, skin cancer, cancer of the brain, cervical cancer, intestinal cancer, liver cancer, colon cancer, stomach cancer, intestine cancer, gastrointestinal cancer, lymph node cancer, esophagus cancer, colorectal cancer, pancreas cancer, ear, nose and throat (ENT) cancer, cancer of the uterus, ovarian cancer and lung cancer.

20. The use of claim 19, wherein said fusion construct is locally administered as anti-tumour adjuvant for the treatment of residual cancer after surgery in breast cancer patients and patients with glioblastoma (astrocytoma VI).

21. The use of claim 19 or 20, wherein said fusion construct is administered at a concentration of 0,5 to 5 µg/ml, preferably 1 µg/ml.

22. The use of any one of claims 18-21, wherein said fusion construct modulates leukocyte killing and effector cell function.

23. The use of any one of claims 18-22, wherein said fusion construct results in a recruitment of leukocytes, cytotoxic T-cells, natural killer (NK) cells, or granulocytes to the tumour site.

24. An *in vitro* method for inhibition of cancer cell proliferation comprising the steps
(1) subjecting a cancer cell line to an effective amount of chemokine-mucin-GPI construct.

25. Use of a polypeptide of a chemokine-mucin-GPI according to any one of claims 1 to 12 for the preparation of a medicament for the treatment of acute vascular damage during allograft transplantation and/or for use in tissue regeneration.

26. The use of claim 25, wherein the GPI-anchored fusion constructs is incorporated in the surface membrane of target cells and modulates angiogenesis.

## Patentansprüche

1. Chemokin-Fusionskonstrukt umfassend eine Aminosäuresequenz eines Chemokins oder eines biologisch aktiven Fragmentes davon, wobei das Chemokin oder biologisch aktive Fragment davon mit einer Mucindomäne gefolgt von einem Glycosylphosphatidylinositol (GPI)-Anker verbunden ist.

2. Fusionskonstrukt von Anspruch 1, wobei die Mucindomäne eine Membran gebundene Mucindomäne ist.

3. Fusionskonstrukt von Anspruch 2, wobei die Mucindomäne eine Aminosäuresequenz umfasst, die durch ein Gen kodiert wird, ausgewählt aus der Gruppe bestehend aus MUC1, MUC3A, MUC3B, MUC4, MUC11, MUC12, MUC16 und MUC17, oder einem Teil davon.

4. Fusionskonstrukt von Anspruch 1, wobei die Mucindomäne einen Mucinstiel des oberflächenassoziierten Chemokins CXCL16 oder Fractalkins (CX3CL1) ist.

5. Fusionskonstrukt von jedem der Ansprüche 1-4, wobei das Chemokin ein Mitglied der C, CC, CXC oder CX₃C Subfamilie von Chemokinen ist.

6. Fusionskonstrukt von Anspruch 5, wobei die Chemokin C Subfamilie ausgewählt ist aus der Gruppe bestehend aus XCL1/Lymphotactin/SCM-1a und XCL2/SCM-1β.

7. Fusionskonstrukt von Anspruch 5, wobei die Chemokin CC Subfamilie ausgewählt ist aus der Gruppe bestehend aus CCL1/I-309, CCL2/MCP-1, CCL3/MIP-1α, CCL3L1/LD78β,CCL4/MIP-1β,CCL5/RANTES, CCL6, CCL7/MCP3, CCL8/MCP-2, CCL9/CCL10, CCL11/Eotaxin, CCL12, CCL13/MCP-4, CCL14/HCC-1, CCL15/HCC-2/Lkn-1/MIP-1δ, CCL16/HCC-4/LEC/LCC-1, CCL17/TARC, CCL18/DC-CK1; CCL19/MIP-3β/ELC, CCL20/MIP-3α/LARC, CCL21/6Ckine/SLC, CCL22/MDC, CCL23/MPIF-1/CKb8, CCL24/Eotaxin-2, CCL25/TECK, CCL26/Eotaxin-3, CCL27/CTACK, CCL28/MEC.

8. Fusionskonstrukt von Anspruch 5, wobei die Chemokin CXC Subfamilie ausgewählt ist aus der Gruppe bestehend aus CXCL1/GROα, CXCL2/GROβ, CXCL3/GROγ, CXCL4/PF4, CXCL5/ENA-78, CXCL6/GCP-2, CXCL7/NAP-2, CXCL8/IL-8, CXCL9/Mig, CXCL10/IP-10, CXCL11/I-TAC, CXCL12/SDF-1α/β, CXCL13BCA-1, CXCL14/BRAK, CXCL15, CXCL 16.

9. Fusionskonstrukt von jedem der Ansprüche 1-5, wobei das Chemokin ausgewählt ist aus der Gruppe bestehend aus Fractalkin/CX₃CL1, CXCL16, IL-8/CXCL8, IL-10, IP-10/CXCL10, RANTES/CCL5.

10. Fusionskonstrukt von jedem der Ansprüche 1-10, wobei das Fusionskonstrukt eine oder mehrere GIP-Signalsequenzen enthält, um die GPI-Verankerung zu leiten.

11. Fusionskonstrukt von jedem der Ansprüche 1-10, wobei der GPI-Anker abgeleitet ist von dem Lymphozyten funktionsassoziiertem Antigen (LFA-3) oder einem Teil davon.

12. Fusionskonstrukt von jedem der Ansprüche 1-11, wobei das Chemokin-Mucin-GPI Konstrukt sich in die Zellmembranen von Zielzellen eingefügt.

13. Nukleinsäuremolekül umfassend eine Nukleinsäuresequenz, die für das Fusionskonstrukt gemäß jedem der Ansprüche 1-12 kodiert.

14. Expressionsplasmid umfassend das Nukleinsäuremolekül gemäß Anspruch 13 und zusätzliche Expressionselemente.

15. Wirtszelle umfassend das Expressionsplasmid gemäß Anspruch 14.

16. Vektor umfassend das Nukleinsäuremolekül gemäß Anspruch 13.

17. Pharmazeutische Zusammensetzung umfassend das Fusionskonstrukt gemäß jedem der Ansprüche 1-12 oder des Nukleinsäuremoleküls gemäß Anspruch 13 und einen pharmazeutisch verträglichen Träger.

18. Verwendung eines Polypeptids eines Fusionskonstrukts gemäß jedem der Ansprüche 1-12 zur Herstellung eines Medikamentes zur Behandlung von Krebs.

19. Verwendung gemäß Anspruch 18, wobei der Krebs ausgewählt ist aus der Gruppe bestehend aus Brustkrebs, renalem Krebs, Prostatakrebs, Seminomen, Melanomen , Teratomen, Neuroblastomen, Gliomen, Rektalkrebs, Endometrialem Krebs, Nierenkrebs, Adrenalkrebs, Thyroidkrebs, Blutkrebs, Hautkrebs, Krebs des Gehirns, Zervikalkrebs, Intestinalkrebs, Leberkrebs, Kolonkrebs, Magenkrebs, intestinalem Krebs, gastrointestinalem Krebs, Lymphknotenkrebs, ösophagealem Krebs, kolorektalem Krebs, Pankreaskrebs, Ohren-, Nasen- und Rachenkrebs (ENT), Krebs des Uterus, ovarialem Krebs und Lungenkrebs.

20. Verwendung von Anspruch 19, wobei das Fusionskonstrukt lokal als Anti-Tumor Adjuvanz zur Behandlung von Residualkrebs in Brustkrebspatienten und Patienten mit Glioblastomen (Astrocytoma VI) verabreicht wird.

21. Verwendung von Ansprüchen 19 oder 20, wobei das Fusionskonstrukt bei einer Konzentration von 0,5 bis 5 µg/ml, vorzugsweise 1 µg/ml verabreicht wird.

22. Verwendung von jedem der Ansprüche 18-21, wobei das Fusionskonstrukt das Töten von Leukozyten und Effektorzellfunktion moduliert.

23. Verwendung von jedem der Ansprüche 18-22, wobei das Fusionskonstrukt in der Rekrutierung von Leukozyten, zytotoxischen T-Zellen, Natürlichen Killerzellen (NK), oder Granulozyten an die Tumorstelle resultiert.

24. *In-vitro* Verfahren zur Hemmung von Krebszellproliferation umfassend die folgenden Schritte:
a. Eine Krebszelllinie einer wirksamen Menge Chemokin-Mucin-GPI Fusionskonstrukt aussetzen.

25. Verwendung eines Polypeptids eines Chemokin-Mucin-GPI gemäß jedem der Ansprüche 1 bis 12 zur Herstellung eine Medikamentes zur Behandlung eines akuten Gefäßschadens während einer Allograft-Transplantation und/oder zur Verwendung in der Gewebsregeneration.

26. Verwendung von Anspruch 25, wobei das GPI-verankerte Fusionskonstrukt in die Oberflächenmembran der Zielzellen inkorporiert ist und die Angiogenese moduliert.

## Revendications

1. Une construction de fusion de chimiokines comprenant une séquence d'acides aminés d'une chimiokine ou d'un fragment biologiquement actif d'une chimiokine, où ladite chimiokine ou le fragment biologiquement actif d'une chimiokine est lié à un domaine de mucine suivi par un ancre de glycosylphosphatidylinositole (GPI).

2. La construction de fusion selon la revendication 1, où ledit domaine de mucine est un domaine de mucine lié à une membrane.

3. La construction de fusion selon la revendication 2, où ledit domaine de mucine comprend une séquence d'acides aminés codée par un gène sélectionné parmi le groupe composé de MUC1, MUC3A, MUC3B, MUC4, MUC11, MUC12, MUC16 et MUC17 ou des parties de ces mucines.

4. La construction de fusion selon la revendication 1, où ledit domaine de mucine comprend une trabécule de mucine de la chimiokine associée à la surface CXCL16 ou de la fractalkine (CX3CL1).

5. La construction de fusion selon une quelconque des revendications 1 à 4, où ladite chimiokine est un membre de la sous-famille C, CC, CXC ou CX₃C de chimiokines.

6. La construction de fusion selon la revendication 5, où ladite sous-famille C de chimiokines est sélectionnée parmi le groupe composé de XCL1/Lymphotactine/SCM-1a, et XCL2/SCM-1β.

7. La construction de fusion selon la revendication 5, où ladite sous-famille CC de chimiokines est sélectionnée parmi le groupe composé de CCL1/I-309, CCL2/MCP-1, CCL3/MIP-1α, CCL3L1/LD78β, CCL4/MIP-1β, CCL5/RANTES, CCL6, CCL7/MCP3, CCL8/MCP-2, CCL9/CCL10, CCL11/éotaxine, CCL12, CCL13/MCP-4, CCL14/HCC-1, CCL15/HCC-2/Lkn-1/MIP-1δ, CCL16/HCC-4/LEC/LCC-1, CCL17/TARC, CCL18/DC-CK1, CCL19/MIP-3β/ELC, CCL20/MIP-3α/LARC, CCL21/6Ckine/SLC, CCL22/MDC, CCL23/MPIF-1/CKb8, CCL24/éotaxine-2, CCL25/TECK, CCL26/éotaxine-3, CCL27/CTACK, CCL28/MEC.

8. La construction de fusion selon la revendication 5, où ladite sous-famille CXC de chimiokines est sélectionnée parmi le groupe composé de CXCL1/GROα, CXCL2/GROβ, CXCL3/GROγ, CXCL4/PF4, CXCL5/ENA-78, CXCL6/GCP-2, CXCL7/NAP-2, CXCL8/IL-8, CXCL9 /Mig, CXCL10/IP-10, CXCL11/I-TAC, CXCL12/SDF-1α/β, CXCL13/BCA-1, CXCL14/BRAK, CXCL15, CXCL16.

9. La construction de fusion selon une quelconque des revendications 1 à 5, où ladite chimiokine est sélectionnée parmi le groupe composé de fractalkine/CX₃CL1, CXCL16, IL-8/CXCL8, IL-10, IP-10/CXCL10, RANTES/CCL5.

10. La construction de fusion selon une quelconque des revendications 1 à 9, où ladite construction de fusion comporte une ou plus de séquences de signal GPI pour diriger l'ancrage de GPI.

11. La construction de fusion selon une quelconque des revendications 1 à 10, où l'ancre GPI est dérivé de l'antigène associé à la fonction de lymphocyte (LFA-3) ou une partie de cet antigène.

12. La construction de fusion selon une quelconque des revendications 1 à 11, où ladite construction de chimiokine - mucine - GPI est incorporée dans les membranes cellulaires des cellules cibles.

13. Une molécule d'acide nucléique comprenant une séquence d'acide nucléique qui code pour la construction de fusion selon n'importe laquelle des revendications 1 à 12.

14. Un plasmide d'expression comprenant la molécule nucléique selon la revendication 13 et des éléments d'expression supplémentaires.

15. Une cellule hôte comprenant le plasmide d'expression selon la revendication 14.

16. Un vecteur comprenant une molécule d'acide nucléique selon la revendication 13.

17. Une composition pharmaceutique comprenant la construction de fusion selon une quelconque des revendications 1 à 12, ou la molécule d'acide nucléique selon la revendication 13, et un vecteur acceptable du point de vue pharmaceutique.

18. Utilisation d'un polypeptide d'une construction de fusion selon n'importe laquelle des revendications 1 à 12 pour la préparation d'un médicament pour le traitement du cancer.

19. Utilisation selon la revendication 18, où ledit cancer est sélectionné parmi le groupe composé du cancer du sein, du cancer rénal, du cancer de la prostate, des séminomes, des mélanomes, des tératomes, des neuroblastomes, des gliomes, du cancer du rectum, du cancer de l'endomètre, du cancer du rein, du cancer surrénal, du cancer de la thyroïde, de la leucémie, du cancer de la peau, du cancer du cerveau, du cancer cervical, du cancer des intestins, du cancer du foie, du cancer du colon, du cancer de l'estomac, du cancer de l'intestin, du cancer gastro-intestinal, du cancer des noeuds lymphoïdes, du cancer de l'oesophage, du cancer colorectal, du cancer du pancréas, du cancer otorhinolaryngologique (ENT), du cancer de l'utérus, du cancer des ovaires et du cancer des poumons.

20. L'utilisation selon la revendication 19, où ladite construction de fusion est localement administrée comme adjuvent anticancéreux pour le traitement du cancer résiduel après une intervention chirurgicale chez un patient souffrant d'un cancer des seins et des patients avec un glioblastome (astrocytome VI).

21. L'utilisation selon la revendication 19 ou 20, où ladite construction de fusion est administrée à une concentration entre 0,5 et 5 µg / ml, de préférence 1 µg / ml.

22. L'utilisation selon n'importe laquelle des revendications 18 à 21, où ladite construction de fusion module la suppression de leucocytes et la fonction de cellule effectrice.

23. L'utilisation selon n'importe laquelle des revendications 18 à 22, où ladite construction de fusion génère un repeuplement de leucocytes, des cellules T cytotoxiques, des cellules tueuses naturelles (NK) , ou des granulocytes au site de la tumeur.

24. Un procédé *in vitro* pour l'inhibition de la prolifération des cellules cancéreuses comprenant les étapes (1) d'exposition d'une ligne de cellules cancéreuses dans une quantité suffisante à la construction de chimiokine - mucine - GPI.

25. Utilisation d'un polypeptide de chimiokine - mucine - GPI selon n'importe laquelle des revendications 1 à 12 pour la préparation d'un médicament pour le traitement d'un dommage vasculaire aigu pendant la transplantation allogreffe et / ou pour l'utilisation dans la régénération de tissu.

26. L'utilisation selon la revendication 25, où la construction de fusion ancrée à GPI est incorporée dans la membrane de surface des cellules cibles et modulent l'angiogénèse.
